# EUROPEAN PATENT APPLICATION

(11) **EP 4 296 265 A1**
(43) Date of publication of application: **27.12.2023**
(21) Application number: 23176278.2
(22) Date of filing: 31.05.2023
(51) Int. Cl.: C07D 213/26, C07D 209/58, C07D 209/86, C07D 219/02, C07D 219/14, C07D 241/46, C07D 265/38

(54) **USE OF A CATALYST COMPOSITION FOR BIARYL SYNTHESIS BY DECARBOXYLATIVE CROSS-COUPLING**

(30) Priority: 21.06.2022 US 202263366714 P
(71) Applicant: Heraeus Deutschland GmbH & Co. KG, 63450 Hanau (DE); New Iridium, Inc., Boulder, CO 80303-1961 (US)
(72) Inventor: GOCK, Michael, 63450 Hanau (DE); WALTER, Philipp, 63450 Hanau (DE); KOLVENBACH, Robin, 63450 Hanau (DE); RILEY, Paige, Boulder, 80303-1961 (US); ELDER, William Zachary, Boulder, CO 80303-1961 (US); QIAN, Gang, Boulder, CO 80303-1961 (US); LIM, Chern-Hooi, Boulder, CO 80303-01961 (US)
(74) Representative: Heraeus IP

(57) **Abstract**

The present invention relates to a process for coupling a heterocyclic aromatic ring AR1 and a carbocyclic or heterocyclic aromatic ring AR2 to each other by a light-assisted decarboxylative carbon-carbon cross-coupling reaction, wherein
- a reaction medium is provided by mixing a first reactant, a second reactant and a catalyst composition, wherein the catalyst composition comprises
(i) a palladium compound which is a palladium salt or a palladium complex or a mixture thereof, and
(ii) a polycyclic compound of Formula (I), (II) or (III):

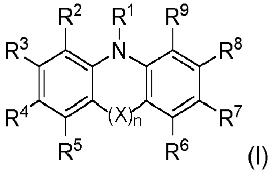

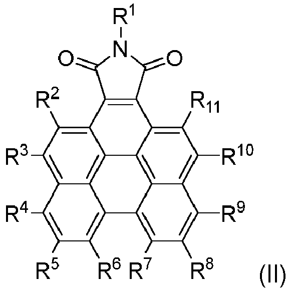

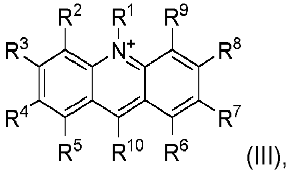

- the reaction medium is irradiated by an external light source, thereby coupling the heterocyclic aromatic ring AR1 of the first reactant to the aromatic ring AR2 of the second reactant by a decarboxylative carbon-carbon cross-coupling reaction.

## Description

### TECHNICAL FIELD

The invention relates to a process for preparing heteroaromatic biaryl compounds by a light-assisted decarboxylative carbon-carbon cross-coupling reaction using a specific catalyst composition.

### BACKGROUND

Biaryl structures, especially those containing heterocycles, form the core of many polymers, ligands, biologically active and functional molecules. Accordingly, efficient synthesis methods for preparing biaryl compounds are needed.

Several types of (hetero)aryl-(hetero)aryl cross-coupling methods have been developed for the synthesis of biaryl structures using different carbon nucleophiles, namely Suzuki (organoboron reagents), Stille (organotin reagents), Negishi (organozinc reagents), and Kumada (Grignard reagents) cross-coupling reactions.

Cross-coupling reactions according to Stille, Negishi and Kumada have several disadvantages compared to Suzuki. The drawbacks of Stille reactions are due to the use of organotin reagents, which are highly toxic, costly and have lower functional group tolerance. Negishi reaction tends to result in lower yields, less functional group tolerance and is water and oxygen sensitive. Due to the high reactivity of the Grignard reagent, Kumada couplings have limited functional group tolerance and are prone to protonolysis from even mildly acidic groups, which can be problematic in large syntheses. Accordingly, Suzuki has become the preferred method used in the industry due to its milder conditions, high tolerance toward functional groups, use of less toxic and more stable boron-based nucleophiles, ease of handling and purification from its reaction mixtures.

Despite the advantages and benefits of Suzuki reaction for biaryl synthesis compared to other methods, Suzuki still suffers from fundamental drawbacks, which limit its application in heterocyclic substrates and base-sensitive substrates.

For example, some (hetero)aryl boronic acids may quickly deboronate under basic conditions and are thus challenging and problematic coupling partners. Furthermore, high catalyst loading is frequently needed.

Because of the drawbacks and limited substrates of Suzuki coupling (e.g. difficult preparation of boron reagents, unstable (hetero)aryl boronic acid and use of stoichiometric amounts of expensive organometallic compounds), there are significant benefits in developing an alternative and superior method for the synthesis of biaryls.

An alternative approach for preparing biaryl compounds is by decarboxylative carbon-carbon cross-coupling (see e.g. a review of L. Liu et al., "Transition metal-catalyzed decarboxylative cross-coupling reactions", Sci China Chem, Vol. 54, 2011, pp. 1670-1687).

Nilsson et al., "A new biaryl synthesis illustrating a connection between the Ullmann biaryl synthesis and copper-catalyzed decarboxylation", Acta Chem. Scand., 1966, 20 (2), pp. 423-426, describe the biaryl synthesis by copper catalyzed decarboxylation of aromatic acid at 240°C. The drastic conditions and intrinsic limitations of this copper-catalyzed Ullmann cross-coupling reaction preclude the preparative application of this reaction.

Liu et al., "Copper-Catalyzed Decarboxylative Cross-Coupling of Potassium Polyfluorobenzoates with Aryl Iodides and Bromides", Angewandte Chemie International Edition 2009, 48 (49), 9350-9354, describe a copper-catalyzed reaction with lower temperature of about 130°C. However, the substrate is limited to perfluorinated aromatic acid.

Palladium-catalyzed carbon-carbon cross-coupling of heteroaromatic carboxylic acids is described by the following publications:
- P. Forgione et al., "Unexpected intermolecular Pd-catalyzed cross-coupling reaction employing heteroaromatic carboxylic acids as coupling partners", Journal of the American Chemical Society, 2006, 128 (35), 11350-11351;
- S. Messaoudi et al., "Palladium-catalyzed decarboxylative coupling of quinolinone-3-carboxylic acids and related heterocyclic carboxylic acids with (hetero) aryl halides", Organic Letters, 2012, 14 (6), 1496-1499;
- F. Bilodeau et al., "Palladium-catalyzed decarboxylative cross-coupling reaction between heteroaromatic carboxylic acids and aryl halides", The Journal of Organic Chemistry, 2010, 75 (5), 1550-1560;
- F. Arroyave et al., "3,4-Propylenedioxypyrrole-based conjugated oligomers via Pd-mediated decarboxylative cross coupling", Organic Letters, 2010, 12 (6), 1328-1331;
- M. Miyasaka et al., "Fluorescent diarylindoles by palladium-catalyzed direct and decarboxylative arylations of carboxyindoles", Chemistry, 2009, 15 (15), 3674-7.

As opposed to a monometallic catalyst system, the use of Cu/Pd-based bimetallic catalysts for the synthesis of biaryls through decarboxylative carbon-carbon cross-coupling of (hetero)aryl carboxylic acids and (hetero)aryl halides or sulfonates has been described by the following publications:
- L.J. Gooßen et al., "Synthesis of biaryls via catalytic decarboxylative coupling", Science, 2006, 313 (5787), 662-4;
- L.J. Gooßen et al., "Biaryl synthesis via Pd-catalyzed decarboxylative coupling of aromatic carboxylates with aryl halides", Journal of the American Chemical Society, 2007, 129 (15), 4824-4833;
- L.J. Gooßen et al., "Palladium/copper-catalyzed decarboxylative cross-coupling of aryl chlorides with potassium carboxylates", Angewandte Chemie International Edition 2008, 47 (37), 7103-7106;
- D. Hackenberger et al., "Synthesis of 3-Substituted 2-Arylpyridines via Cu/Pd-Catalyzed Decarboxylative Cross-Coupling of Picolinic Acids with (Hetero)Aryl Halides", J. Org. Chem. 2017, 82 (7), 3917-3925;
- L.J. Gooßen et al., "Decarboxylative biaryl synthesis from aromatic carboxylates and aryl triflates", Journal of the American Chemical Society, 2008, 130 (46), 15248-15249;
- D. Hackenberger et al., "Bimetallic Cu/Pd Catalysts with Bridging Aminopyrimidinyl Phosphines for Decarboxylative Cross-Coupling Reactions at Moderate Temperature", ChemCatChem 2015, 7 (21), 3579-3588;
- L.J. Gooßen et al., "Decarboxylative Cross-Coupling of Aryl Tosylates with Aromatic Carboxylate Salts", Angewandte Chemie, 2010, 122 (6), 1129-1132;
- L.J. Gooßen et al., "Decarboxylative cross-coupling of mesylates catalyzed by copper/palladium systems with customized imidazolyl phosphine ligands", Angew. Chem. Int. Ed. Engl. 2013, 52 (10), 2954-8;
- L.J. Gooßen et al., "Catalytic Decarboxylative Cross-Coupling of Aryl Chlorides and Benzoates without Activating ortho Substituents", Angew. Chem. Int. Ed. Engl. 2015, 54 (44), 13130-3.

F. Bilodeau et al, J. Org. Chem., 2010, 75 (5), pp. 1550-60, describe a method wherein heteroaryl carboxylates are coupled with various aryl halides to form corresponding arylated heteroarenes by a monometallic Pd catalyst system, instead of a bimetallic catalyst system. The reaction occurred at 170°C under microwave irradiation.

However, high reaction temperatures are very unfavorable since many pharmaceutical compounds tend to change their stereochemistry or even decompose when heated this high.

Recently, light-induced photoredox catalysis has become a tool for the design and development of a variety of radical reactions. A review on photoredox catalysis in organic chemistry is provided by M.H. Shaw et al., J. Org. Chem., 2016, 81, pp. 6898-6926.

US 10,975,171 B2 discloses phenoxazine and dihydrophenazine photoredox catalysts which might be used in atom transfer radical addition/polymerization, dehalogenation, cycloaddition, cyclization, dimerization, coupling, reduction, ring-opening, alkylation, arylation, oxygenation and radical addition. As indicated in US 10,975,171 B2, the reaction medium is preferably free of a metal or metalloid.

Dimethyl dihydroacridines as photoredox catalysts in atom transfer radical polymerization of acrylate monomers is described by C. Lim et al., Angew. Chem. Int. Ed. Engl., 2020, 59(8), pp. 3209-3217.

C. Lim et al., Chem. Eur. J., 2017, 23(46), pp. 10962-10968, describe strongly reducing visible light organic photoredox catalysts (such as N-aryl phenoxazines and dihydrophenazines) as an alternative to iridium and ruthenium photoredox catalysts.

C. Lim et al, J. Am. Chem. Soc., 2020, 142, 31, pp. 13573-13581, describe a light-induced Birch reduction in the presence of a benzoperylene monoimide acting as a photoredox catalyst.

A combination of transition metal catalysts and photoredox catalysts is described by J. Twilton et al., "The merger of transition metal and photocatalysis", Nat Rev Chem, 1, 0052 (2017), https://doi. org/10.1038/s41570-017-0052.

A review on cooperative photoredox and palladium catalysis is provided by N.D.P. Singh et al., Catal. Sci. Technol., 2021, 11, pp. 742-767.

It remains very challenging to prepare heteroaromatic biaryl compounds (i.e. biaryl compounds wherein at least one of the two aromatic rings being linked to each other is a heterocyclic aromatic ring) under relatively moderate reaction conditions.

Accordingly, an object of the present invention is to provide a process by which a heterocyclic aromatic ring and a carbocyclic or heterocyclic aromatic ring can be coupled to each other (in particular for preparing heteroaromatic biaryl compounds) even at rather moderate temperatures (e.g. less than 100°C). It is also an object of the present invention to provide a composition which can be used as a catalyst in decarboxylative cross-coupling reactions for coupling a heterocyclic aromatic ring and a carbocyclic or heterocyclic aromatic ring to each other.

### SUMMARY

The invention provides a process which comprises
- mixing a first reactant, a second reactant and a catalyst composition, thereby providing a reaction medium, wherein
   the first reactant is a compound which comprises a heterocyclic aromatic ring AR1 to which a carboxylic acid group, a carboxylate group or a carboxylic acid ester group is attached,
   the second reactant is a compound which comprises an aromatic ring AR2 to which a halogen atom (e.g. Cl, Br or I), a pseudohalogen group (e.g. cyano, cyanate or isocyanate) or a sulfonate group (such as a trifluoromethanesulfonate group -O-Tf) is attached,
   the catalyst composition comprises
      (i) a palladium compound which is selected from a palladium salt or a palladium complex or a mixture thereof,
      (ii) a polycyclic compound of Formula (I), (II) or (III): 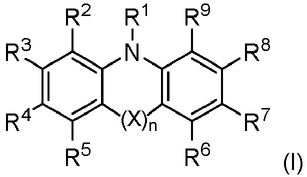 wherein in Formula (I)
         n is 0 or 1,
         X is O, C(R¹⁰)(R¹¹), or N(R¹²),
         R' to R¹² are selected independently from each other from hydrogen, aryl and C₁₋₁₀alkyl, wherein aryl and C₁₋₁₀alkyl are optionally substituted with one or more substituents R¹³, wherein the one or more substituents R¹³, if present, are selected independently from each other from C₁₋₆alkyl, C₁₋₆haloalkyl, -OH, -O-(C₁₋₆alkyl), -NO₂, -CN, halogen (e.g. F, Cl, Br or I), -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 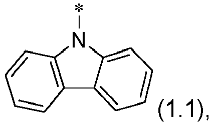 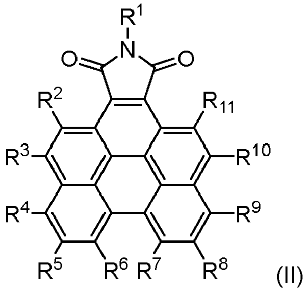
         wherein in Formula (II)
         R' is selected from C₁₋₁₆alkyl and aryl, each of which is optionally substituted with one or more substituents R¹², and/or C₁₋₁₆alkyl is optionally interrupted by one or more oxygen atoms;
         R² to R¹¹ are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms; or at least two of the residues R² to R¹¹ which are adjacent to each other form together with the carbon atoms to which they are attached a five- or six-membered aromatic ring and, if present, the remaining residues (i.e. those of the residues R² to R¹¹ which do not form a ring) are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms;
         wherein the one or more substituents R¹², if present, are selected independently from each other from C₁₋₆alkyl, C₁₋₆haloalkyl, -OH, -O-(C₁₋₆alkyl), -NO₂, -CN, halogen (e.g. F, Cl, Br or I), -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 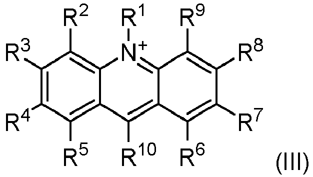
         wherein in Formula (III)
         R' is selected from C₁₋₁₆alkyl and aryl, each of which is optionally substituted with one or more substituents R¹¹, and/or C₁₋₁₆alkyl is optionally interrupted by one or more oxygen atoms;
         R² to R⁹ are selected independently from each other from hydrogen, C₁₋₁₆alkyl and aryl, wherein C₁₋₁₆alkyl and aryl are optionally substituted with one or more substituents R¹¹, and/or C₁₋₁₆alkyl is optionally interrupted by one or more oxygen atoms;
         R¹⁰ is aryl which is optionally substituted with one or more substituents R¹²,
         wherein the one or more substituents R¹¹, if present, are selected independently from each other from C₁₋₆alkyl, C₁₋₆haloalkyl, -OH, -O-(C₁₋₆alkyl), -NO₂, -CN, halogen (e.g. F, Cl, Br or I), -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl),
         wherein the one or more substituents R¹², if present, are selected independently from each other from C₁₋₆alkyl and aryl (e.g. phenyl), wherein aryl is optionally substituted with one or more C₁₋₆alkyl;
- irradiating the reaction medium by an external light source, thereby coupling the heterocyclic aromatic ring AR1 of the first reactant to the aromatic ring AR2 of the second reactant by a decarboxylative carbon-carbon cross-coupling reaction.

In Formula (1.1), ∗- denotes the bond by which the carbazolyl group (i.e. substituent R¹³) is connected to the aryl or C₁₋₁₀alkyl residue (i.e. one of the residues R' to R¹²).

The product obtained by the process is a heteroaromatic biaryl compound, i.e. a compound which comprises a heterocyclic aromatic ring AR1 and an aromatic ring AR2 (which might be a carbocyclic or heterocyclic aromatic ring) being linked to each other by a carbon-carbon single bond. The heterocyclic aromatic ring AR1 originates from the first reactant, and the aromatic ring AR2 originates from the second reactant.

The reaction outlined above represents an intermolecular decarboxylative cross-coupling reaction between a first compound and a second compound. Alternatively, the heterocyclic aromatic ring AR1 and the carbocyclic or heterocyclic aromatic ring AR2 might be coupled to each other by an intramolecular decarboxylative cross-coupling reaction (i.e. each of the reactant molecules containing both the heterocyclic aromatic ring AR1 and the carbocyclic or heterocyclic aromatic ring AR2, the rings AR1 and AR2 being linked by a bivalent linker unit).

Accordingly, the present invention also relates to a process which comprises
- mixing a reactant and a catalyst composition, thereby providing a reaction medium, wherein
   the reactant is a compound which comprises a heterocyclic aromatic ring AR1 to which a carboxylic acid group, a carboxylate group or a carboxylic acid ester group is attached and an aromatic ring AR2 to which a halogen atom (Cl, Br or I), a pseudohalogen group (e.g. cyano, cyanate or isocyanate) or a sulfonate group (e.g. trifluoromethanesulfonate group -O-Tf) is attached,
   the catalyst composition comprises
      (i) a palladium compound which is selected from a palladium salt or a palladium complex or a mixture thereof,
      (ii) a polycyclic compound of Formula (I), (II) or (III): 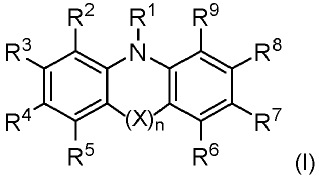 wherein in Formula (I)
         n is 0 or 1,
         X is O, C(R¹⁰)(R¹¹), or N(R¹²),
         R' to R¹² are selected independently from each other from hydrogen, aryl and C₁₋₁₀alkyl, wherein aryl and C₁₋₁₀alkyl are optionally substituted with one or more substituents R¹³, wherein the one or more substituents R¹³, if present, are selected independently from each other from C₁₋₆alkyl, C₁₋₆haloalkyl, -OH, -O-(C₁₋₆alkyl), -NO₂, -CN, halogen (e.g. F, Cl, Br or I), -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 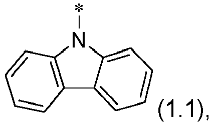 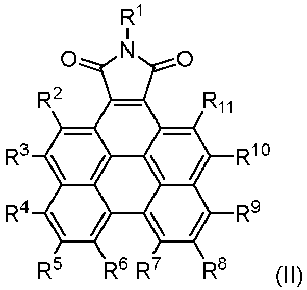
         wherein in Formula (II)
         R' is selected from C₁₋₁₆alkyl and aryl, each of which is optionally substituted with one or more substituents R¹², and/or C₁₋₁₆alkyl is optionally interrupted by one or more oxygen atoms;
         R² to R¹¹ are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms; or
         at least two of the residues R² to R¹¹ which are adjacent to each other form together with the carbon atoms to which they are attached a five- or six-membered aromatic ring and, if present, the remaining residues (i.e. those of the residues R² to R¹¹ which do not form a ring) are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms;
         wherein the one or more substituents R¹², if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 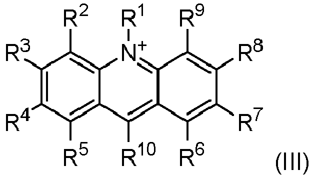
         wherein in Formula (III)
         R¹ is selected from C₁₋₁₆alkyl and aryl, each of which is optionally substituted with one or more substituents R¹¹, and/or C₁₋₁₆alkyl is optionally interrupted by one or more oxygen atoms;
         R² to R⁹ are selected independently from each other from hydrogen, C₁₋₁₆alkyl and aryl, wherein C₁₋₁₆alkyl and aryl are optionally substituted with one or more substituents R¹¹, and/or C₁₋₁₆alkyl is optionally interrupted by one or more oxygen atoms;
         R¹⁰ is aryl which is optionally substituted with one or more substituents R¹²,
         wherein the one or more substituents R¹¹, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl),
         wherein the one or more substituents R¹², if present, are selected independently from each other from C₁₋₆alkyl and aryl (e.g. phenyl), wherein aryl is optionally substituted with one or more C₁₋₆alkyl;
- irradiating the reaction medium by an external light source, thereby coupling the heterocyclic aromatic ring AR1 and the aromatic ring AR2 of the reactant to each other by a decarboxylative carbon-carbon cross-coupling reaction.

The present invention also relates to the use of the composition outlined above and described below in further detail (i.e. the composition comprising (i) a palladium compound and (ii) a polycyclic compound of Formula (I), (II) or (III)) as a catalyst for coupling a heterocyclic aromatic ring AR1 and a carbocyclic or heterocyclic aromatic ring AR2 to each other by a light-assisted decarboxylative carbon-carbon cross-coupling reaction. As already mentioned above, the cross-coupling reaction might be an intermolecular cross-coupling or an intramolecular cross-coupling. The preferred use is for synthesis of biaryl compounds.

### DETAILED DESCRIPTION

As already outlined above, the catalyst composition used in the process of the present invention comprises (i) a palladium compound and (ii) a polycyclic compound of Formula (I), (II) or (III).

If a heteroaryl carboxylic acid or a carboxylate or an ester thereof is reacted with a (hetero)aryl halide (or a (hetero)aryl pseudohalide or a (hetero)aryl sulfonate) in a reaction medium
- to which the catalyst composition of the present invention has been added and
- which is irradiated by an external light source,
a heteroaromatic biaryl compound can be formed by a decarboxylative carbon-carbon cross-coupling reaction even at rather moderate reaction temperature (e.g. 70°C or even less).

However, no heteroaromatic biaryl compound is formed from these reactants at moderate reaction temperature if
- the reaction medium comprises the palladium compound and is irradiated by an external light source but no polycyclic compound of component (ii) is present, or
- the reaction medium comprises both the palladium compound and the polycyclic compound of component (ii) but is not irradiated by an external light source.

As outlined above, the catalyst composition used in the process of the present invention may comprise a polycyclic compound of Formula (I).

If one or more of the residues R¹ to R¹² in Formula (I) are aryl, it can be a monoaryl group (e.g. phenyl or naphthyl) or a biaryl group (e.g. biphenyl).

Preferably, R¹ in Formula (I) is aryl, which can be a monoaryl group (e.g. phenyl or naphthyl) or a biaryl group (e.g. biphenyl).

Preferably, R¹ in Formula (I) is selected from phenyl, naphthyl and biphenyl, each of which is optionally substituted with one or more substituents R¹³, wherein R¹³ has the same meaning as outlined above. Accordingly, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and N-carbazolyl of Formula (1.1)

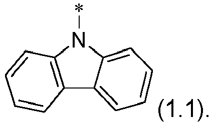

If in Formula (I) n is 0, the polycyclic compound has the following Formula (Ia): 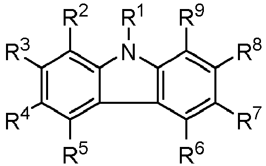 wherein R¹ and R² to R⁹ have the same meanings as outlined above in Formula (I).

In a preferred embodiment of Formula (Ia),
R¹ is selected from naphthyl (e.g. 1-naphthyl or 2-naphthyl), phenyl and biphenyl, each of which is optionally substituted with one or more R¹³;
R² to R⁹ are selected independently from each other from hydrogen, C₁₋₆alkyl, phenyl, biphenyl and naphthyl, wherein phenyl, biphenyl and naphthyl are optionally substituted with one or more substituents R¹³;
wherein, if present, the one or more substituents R¹³, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1)

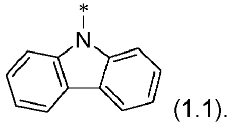

An exemplary compound of Formula (Ia) has the following Formula (I-1):

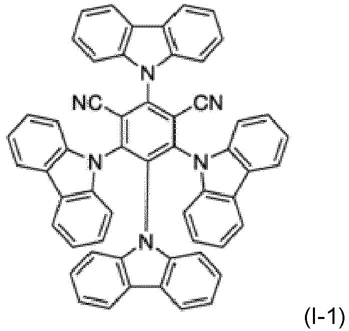

If in Formula (I) n is 1 and X is O, the polycyclic compound has the following Formula (Ib): 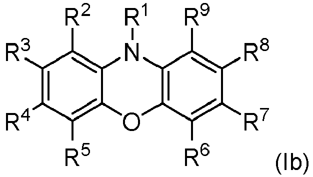 wherein R¹ to R⁹ have the same meanings as outlined above in Formula (I).

In a preferred embodiment of Formula (Ib),
R¹ is naphthyl (preferably 1-naphthyl) which is optionally substituted with one or more substituents R¹³;
R², R³, R⁵, R⁶, R⁸ and R⁹ are selected independently from each other from hydrogen and C₁₋₆alkyl;
R⁴ and R⁷ are selected independently from each other from phenyl, biphenyl and naphthyl, each of which is optionally substituted with one or more substituents R¹³;
wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

Exemplary compounds of Formula (Ib) are those of the following Formulas (I-2), (I-3), (I-4) and (I-5):

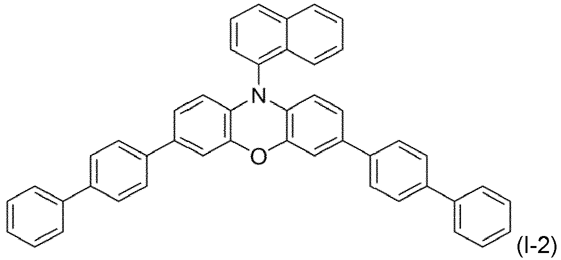

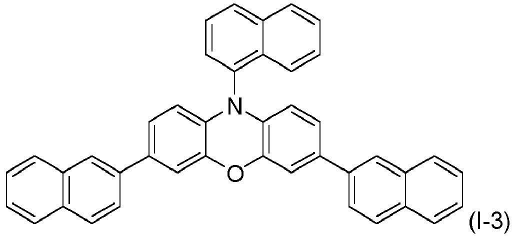

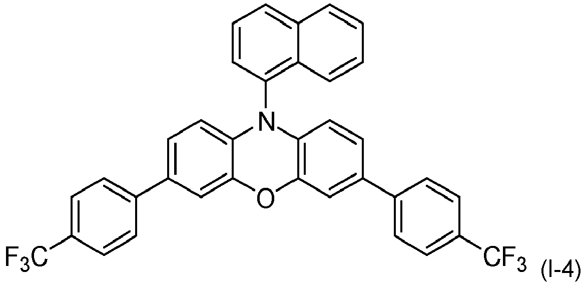

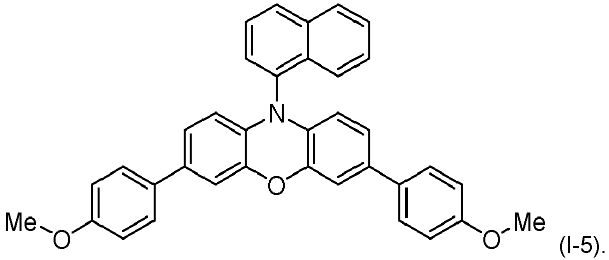

If in Formula (I) n is 1 and X is C(R¹⁰)(R¹¹), the polycyclic compound has the following Formula (Ic): 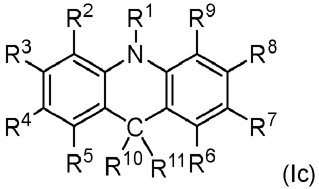 wherein R¹, R² to R⁹ and R¹⁰ to R¹¹ have the same meanings as outlined above in Formula (I).

In a preferred embodiment of Formula (Ic),
R¹⁰ and R¹¹ are selected independently from each other from hydrogen and C₁₋₆alkyl;
R¹ is selected from naphthyl (e.g. 1-naphthyl or 2-naphthyl), phenyl and biphenyl, each of which is optionally substituted with one or more substituents R¹³,
R², R³, R⁵, R⁶, R⁸ and R⁹ are selected independently from each other from hydrogen and C₁₋₆alkyl;
R⁴ and R⁷ are selected independently from each other from phenyl, naphthyl and biphenyl, each of which is optionally substituted with one or more substituents R¹³;
wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl) and -S(O)₂(phenyl).

Exemplary compounds of Formula (Ic) have the following Formulas (I-6.1) to (I-6.7):

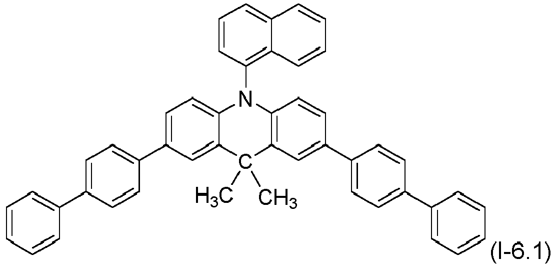

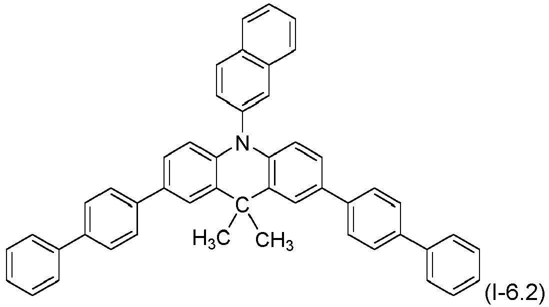

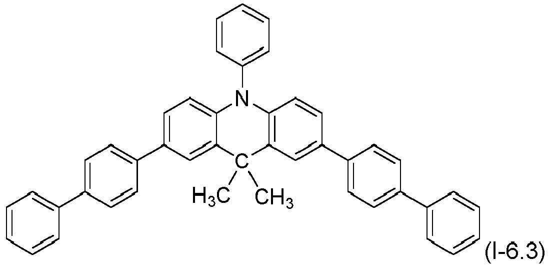

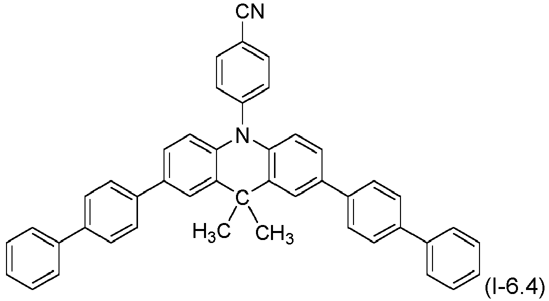

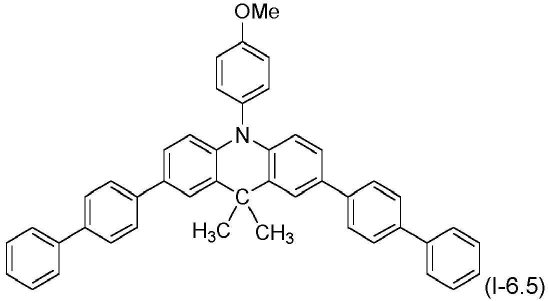

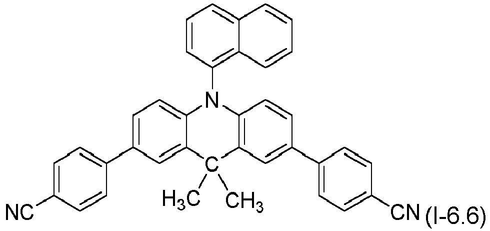

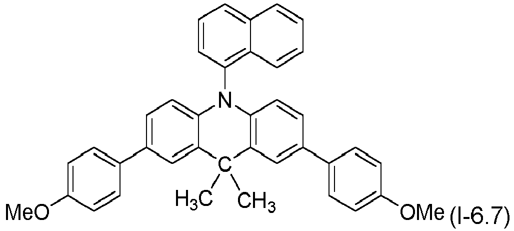

If in Formula (I) n is 1 and X is N(R¹²), the polycyclic compound has the following Formula (Id): 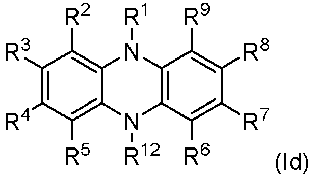 wherein R¹, R² to R⁹ and R¹² have the same meanings as outlined above in Formula (I).

In a preferred embodiment of Formula (Id),
R¹ and R¹² are selected independently from each other from phenyl, naphthyl (such as 2-naphthyl or 1 naphthyl) and biphenyl, each of which is optionally substituted with one or more substituents R¹³;
R² to R⁹ are selected independently from each other from hydrogen and C₁₋₆alkyl; or at least two, preferably all of the residues R³, R⁴, R⁷, and R⁸ are phenyl which is optionally substituted with one or more substituents R¹³ and the residues R², R⁵, R⁶ and R⁹ are selected independently from each other from hydrogen and C₁₋₆alkyl;
wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

Exemplary compounds of Formula (Id) are those of the following Formulas (I-7) and (I-8):

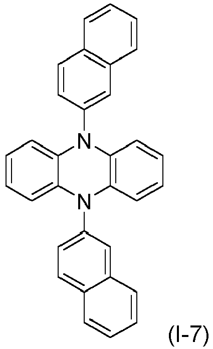

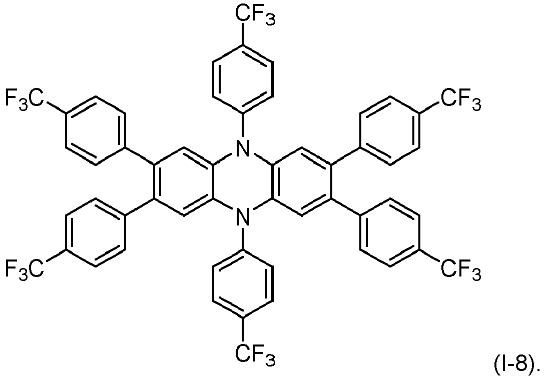

As outlined above, the catalyst composition used in the process of the present invention may comprise a polycyclic compound of Formula (II).

As mentioned above, one or more of the residues R² to R¹¹ in Formula (II) can be heteroaryl. If present, the heteroaryl group preferably comprises or is a five- or six-membered aromatic ring containing one or more (preferably up to three) heteroatoms being selected from oxygen, nitrogen and sulfur. Exemplary heteroaryl groups are pyridinyl, pyridazinyl, pyrimidinyl, pyrazinyl, oxazolyl, thiazolyl, isoxazolyl, isothiazolyl and oxathiazolyl.

In a preferred embodiment of Formula (II),
R¹ is C₁₋₁₂alkyl which is optionally substituted with one or more substituents being selected from -OH, halogen (e.g. F, CI), -NH₂, -NH(C₁₋₆alkyl) and -N(C₁₋₆alkyl)₂;
R⁴, R⁷ and R⁹ are selected independently from each other from hydrogen, aryl (e.g. phenyl), heteroaryl, C₁₋₆alkyl and -O-(C₁₋₆alkyl), wherein aryl and heteroaryl are optionally substituted with one or more substituents which are selected independently from each other from C₁₋₆alkyl, C₁₋₆haloalkyl (e.g. C₁₋₆fluoroalkyl) and -O-(C₁₋₆alkyl);
R², R³, R⁵, R⁶, R⁸, R¹⁰, and R¹¹ are selected independently from each other from hydrogen and C₁₋₆alkyl.

Exemplary compounds of Formula (II) are those of the following Formulas (II-1), (II-2) and (II-3):

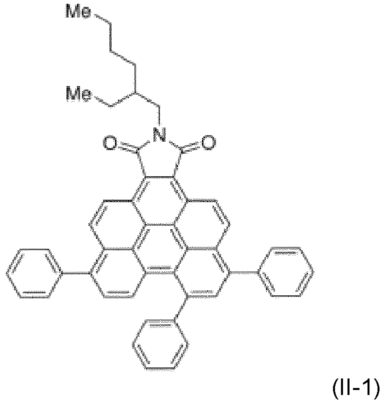

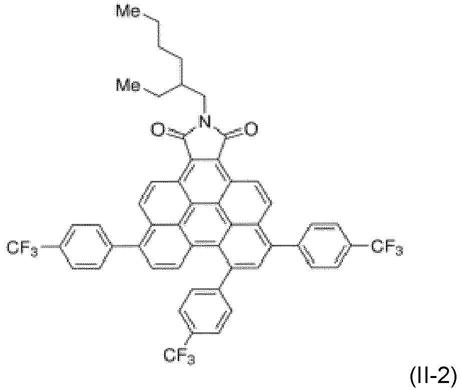

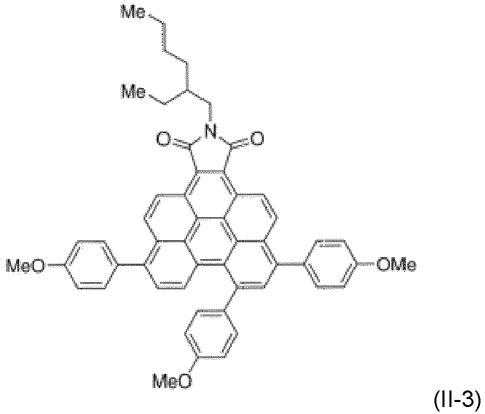

As outlined above, the catalyst composition used in the process of the present invention may comprise a polycyclic compound of Formula (III).

The compound of Formula (III) might be associated with one or more charge-compensating anions, e.g. one or more weakly coordinating anions such as [BF₄]⁻, [ClO₄]⁻, [SbF₆]⁻ and [PF₆]⁻. However, other anions might be present as well.

In a preferred embodiment of Formula (III),
R¹ is C₁₋₆alkyl;
R¹⁰ is phenyl which is optionally substituted with one or more substituents being selected independently from each other from C₁₋₆alkyl, C₁₋₆haloalkyl (e.g. C₁₋₆fluoroalkyl) and -O-(C₁₋₆alkyl);
R² to R⁹ are selected independently from each other from hydrogen and C₁₋₆alkyl.

An exemplary compound of Formula (III) has the following Formula (III-1):

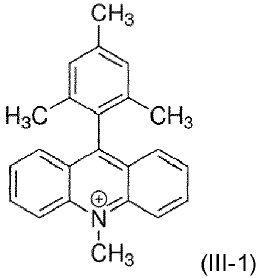

As already outlined above, in addition to the polycyclic compound of component (ii), i.e. the compound of Formula (I), (II) or (III), the catalyst composition used in the process of the present invention comprises a palladium compound (component (i)) which is selected from a palladium salt or a palladium complex or a mixture thereof.

If the palladium compound of component (i) is a palladium salt, it might be a palladium acetate (e.g. Pd(OAc)₂), a palladium acetylacetonate (e.g. Pd(acac)₂) or a palladium halide (such as palladium chloride, palladium bromide or palladium iodide), or any mixture thereof.

If the palladium compound of component (i) is a palladium complex, it might comprise one or more ligands which are selected from a phosphine, a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene or cyclooctatetraene), an amine, or any combination thereof. In addition to one or more of these ligands, the palladium complex may comprise other ligands as well, such as a halide, a pseudohalide (e.g. CN⁻ or OCN⁻), an acetylacetonate or a carboxylate (e.g. acetate).

If the palladium complex of component (i) does not comprise any phosphine ligand, it will be referred to as a phosphine-free palladium complex. The phosphine-free palladium complex may comprise one or more of the non-phosphine ligands mentioned above, i.e. a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene), an amine, or any combination thereof. In addition to one or more of these ligands, the phosphine-free palladium complex may comprise other ligands as well, such as a halide, a pseudohalide (e.g. CN⁻ or OCN⁻), an acetylacetonate or a carboxylate (e.g. acetate). An exemplary phosphine-free palladium complex is a palladium complex comprising one or more ligands of dibenzylideneacetone (e.g. bis(dibenzylideneacetone)palladium or tris(dibenzylideneacetone)dipalladium) or a palladium complex comprising one or more ligands of a cyclic polyene such as cyclooctadiene (COD) or cyclooctatetraene (e.g. Pd(COD)Cl₂).

Optionally, the composition of the present invention may comprise a component (iii) which is selected from a phosphine, a bipyridine or a cyclic diamine or a mixture of at least two of these compounds. Preferably, the composition comprises the compound of component (iii) if the palladium compound of component (i) is a palladium salt or a phosphine-free palladium complex (e.g. a phosphine-free palladium complex comprising one or more ligands selected from dibenzylideneacetone and a cyclic polyene such as cyclooctadiene (COD) or cyclooctatetraene). If the compound of component (iii) is added to the reaction medium together with the palladium compound of component (i), it may subsequently coordinate as a ligand to the palladium atom of the palladium compound.

If the palladium compound of component (i) is a palladium complex which comprises a phosphine as a ligand and the composition further comprises a phosphine compound as component (iii), it is preferred that the phosphine ligand and the phosphine compound of component (iii) are different from each other.

The palladium compound (in particular the palladium salt or the phosphine-free palladium complex) of component (i) and the compound of component (iii) might be kept in separate containers and brought into contact when performing the decarboxylative cross-coupling reaction, thereby forming *in situ* a palladium complex.

According to an exemplary embodiment, the phosphine of component (iii) is of Formula (IV):

P(R¹)(R²)(R³) (IV)

wherein
R¹ and R² are selected independently from each other from C₁₋₆ alkyl and C₅₋₇-cycloalkyl (e.g. cyclohexyl);
R³ is biphenyl which is optionally substituted with one or more R⁴;
R⁴, if present, is C₁₋₆ alkyl, C₁₋₆ alkoxy, phenyl or pyridyl (more preferably C₁₋₆ alkyl or C₁₋₆ alkoxy).

The phosphine of Formula (IV) may have one of the following Formulas (IV-1) to (IV-6): 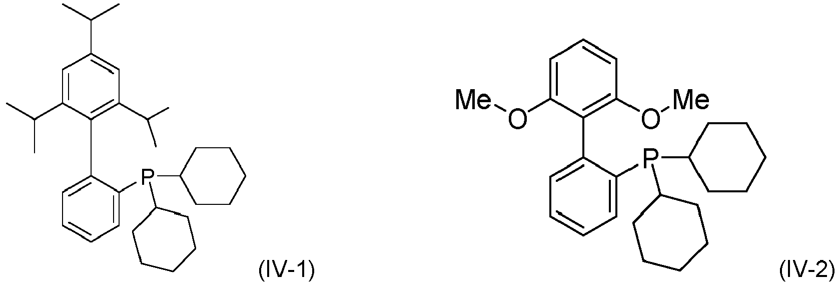 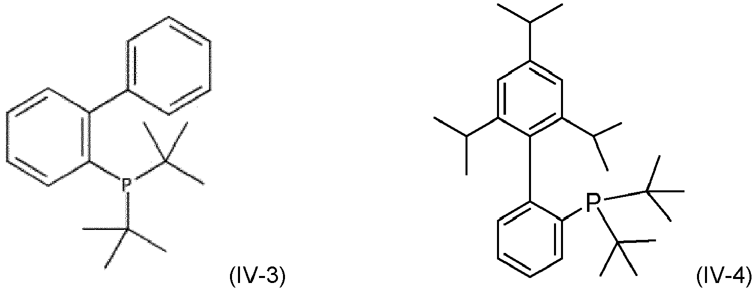 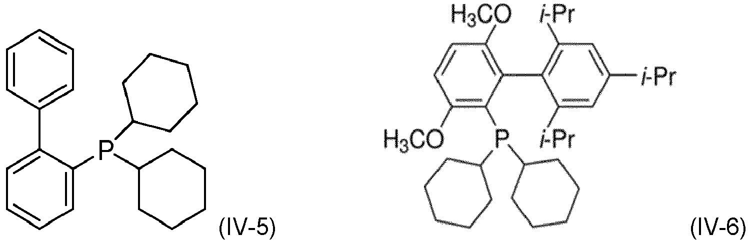 According to another exemplary embodiment, the phosphine of component (iii) is of Formula (V):

P(R¹)(R²)(R³) (V)

wherein
each of R¹ to R³ is C₁₋₆alkyl; or
each of R¹ to R³ is C₅₋₇cycloalkyl (e.g. cyclohexyl); or
R¹ to R³ are selected independently from each other from aryl (e.g. phenyl) and heteroaryl (e.g. pyridyl) wherein each aryl or heteroaryl is optionally substituted with one or more C₁₋₄ haloalkyl (e.g. -CF₃), C₁₋₄ alkyl (e.g. methyl) or C₁₋₄ alkoxy (e.g. methoxy).

The phosphine of Formula (V) may have one of the following Formulas (V-1) to (V-6):

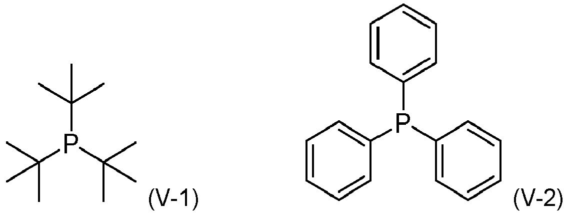

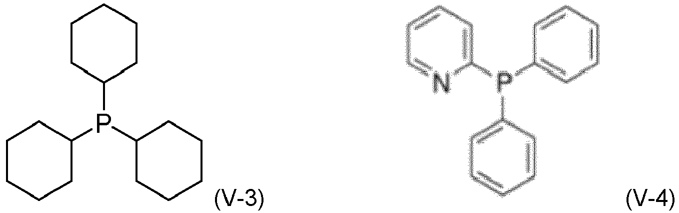

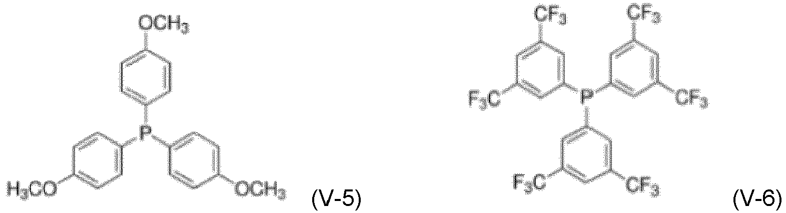

According to another exemplary embodiment, the phosphine of component (iii) is a ferrocenyl diphosphine or a xanthenyl diphosphine.

The ferrocenyl diphosphine may have one of the following Formulas (VI-1) to (VI-2):

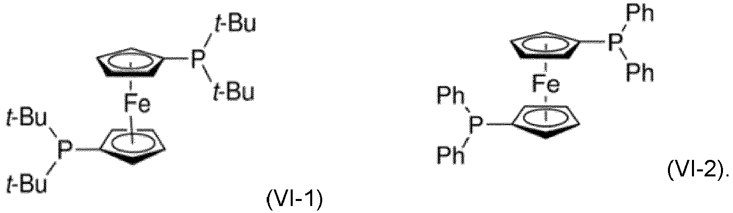

The xanthenyl diphosphine may have the following Formula (VII-1):

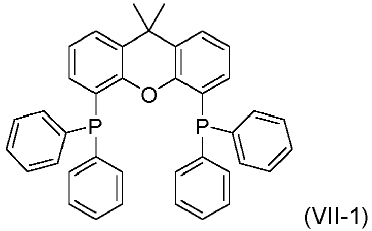

According to another exemplary embodiment, the phosphine of component (iii) is a diphosphine of Formula (VIII):

(R¹)(R²)P-(CH₂)ₙ-P(R³)(R⁴) (VIII)

wherein
n is 1 to 6, more preferably 2 to 4;
R¹, R², R³ and R⁴ are selected independently from each other from phenyl and cyclohexyl, wherein phenyl or cyclohexyl is optionally substituted with one or more C₁₋₄alkyl.

The phosphine of Formula (VIII) may have one of the following Formulas (VIII-1) to (VIII-3):

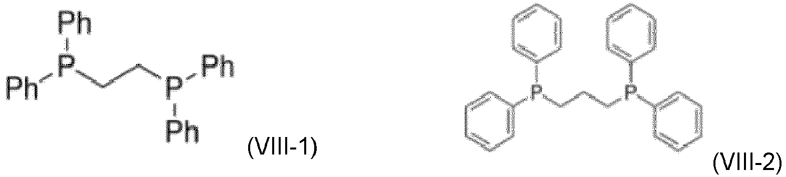

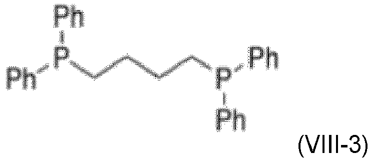

According to another exemplary embodiment, the phosphine of component (iii) is a 2,3-dihydrobenzo[d][1,3]oxaphosphole.

The 2,3-dihydrobenzo[*d*][1,3]oxaphosphole may have the following Formula (IX): 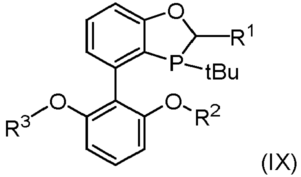 wherein
R¹ to R³ are selected independently from each other from hydrogen and C₁₋₄alkyl.

According to an exemplary embodiment, the 2,3-dihydrobenzo[*d*][1,3]oxaphosphole may have the following Formula (IX-1):

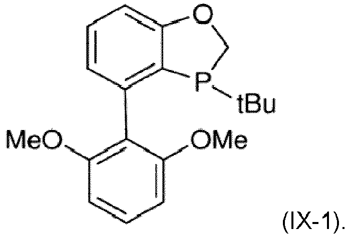

According to another exemplary embodiment, the phosphine of component (iii) is a diadamantyl-C₁₋₆alkyl-phosphine.

The diadamantyl-C₁₋₆alkyl-phosphine may have the following Formula (X-1):

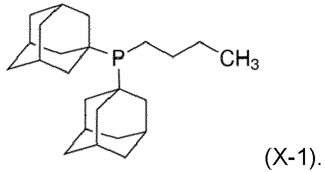

The cyclic diamine of component (iii) is preferably a bicyclic diamine such as 1,4-diazabicyclo[2.2.2]octane (DABCO).

According to an exemplary embodiment of the present invention, the catalyst composition used in the process of the present invention comprises (i) a palladium salt (such as a palladium acetate, a palladium acetylacetonate or a palladium halide) or a phosphine-free palladium complex (e.g. a phosphine-free palladium complex comprising one or more ligands which are selected from a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene) and an amine, and optionally comprising one or more additional ligands which are selected from a halide, a pseudohalide, an acetylacetonate and a carboxylate (e.g. acetate)); (ii) a polycyclic compound of Formula (Ia), and (iii) a phosphine compound as outlined above, preferably a phosphine of Formula (IV). Preferably, the polycyclic compound has the Formula (I-1) outlined above. A preferred phosphine-free palladium complex comprises one or more ligands selected from dibenzylideneacetone and a cyclic polyene such as cyclooctadiene (COD) or cyclooctatetraene. Preferably, the phosphine compound of component (iii) is of Formula (IV-4) indicated above.

According to another exemplary embodiment of the present invention, the catalyst composition used in the process of the present invention comprises (i) a palladium salt (such as a palladium acetate, a palladium acetylacetonate or a palladium halide) or a phosphine-free palladium complex (e.g. a phosphine-free palladium complex comprising one or more ligands which are selected from a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene) and an amine, and optionally comprising one or more additional ligands which are selected from a halide, a pseudohalide, an acetylacetonate and a carboxylate (e.g. acetate)); (ii) a polycyclic compound of Formula (Ib), and (iii) a phosphine compound as outlined above (e.g. a phosphine of Formula (IV)).

According to another exemplary embodiment of the present invention, the catalyst composition used in the process of the present invention comprises (i) a palladium salt (such as a palladium acetate, a palladium acetylacetonate or a palladium halide) or a phosphine-free palladium complex (e.g. a phosphine-free palladium complex comprising one or more ligands which are selected from a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene) and an amine, and optionally comprising one or more additional ligands which are selected from a halide, a pseudohalide, an acetylacetonate and a carboxylate (e.g. acetate)); (ii) a polycyclic compound of Formula (Ic), and (iii) a phosphine compound as outlined above (e.g. a phosphine of Formula (IV)).

According to another exemplary embodiment of the present invention, the catalyst composition used in the process of the present invention comprises (i) a palladium salt (such as a palladium acetate, a palladium acetylacetonate or a palladium halide) or a phosphine-free palladium complex (e.g. a phosphine-free palladium complex comprising one or more ligands which are selected from a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene) and an amine, and optionally comprising one or more additional ligands which are selected from a halide, a pseudohalide, an acetylacetonate and a carboxylate (e.g. acetate)); (ii) a polycyclic compound of Formula (Id), and (iii) a phosphine compound as outlined above (e.g. a phosphine of Formula (IV)).

According to another exemplary embodiment of the present invention, the catalyst composition used in the process of the present invention comprises (i) a palladium salt (such as a palladium acetate, a palladium acetylacetonate or a palladium halide) or a phosphine-free palladium complex (e.g. a phosphine-free palladium complex comprising one or more ligands which are selected from a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene) and an amine, and optionally comprising one or more additional ligands which are selected from a halide, a pseudohalide, an acetylacetonate and a carboxylate (e.g. acetate)); (ii) a polycyclic compound of Formula (II), and (iii) a phosphine compound as outlined above (e.g. a phosphine of Formula (IV)).

According to another exemplary embodiment of the present invention, the catalyst composition used in the process of the present invention comprises (i) a palladium salt (such as a palladium acetate, a palladium acetylacetonate or a palladium halide) or a phosphine-free palladium complex (e.g. a phosphine-free palladium complex comprising one or more ligands which are selected from a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene) and an amine, and optionally comprising one or more additional ligands which are selected from a halide, a pseudohalide, an acetylacetonate and a carboxylate (e.g. acetate)); (ii) a polycyclic compound of Formula (III), and (iii) a phosphine compound as outlined above (e.g. a phosphine of Formula (IV)).

As already indicated above, if the palladium compound of component (i) is a palladium complex, it may comprise one or more ligands which are selected from a phosphine, a 1,4-dien-3-one (e.g. dibenzylideneacetone), an N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene or cyclooctatetraene), an amine, or any combination thereof. In addition to these ligands, the palladium complex may comprise other ligands as well, such as a halide (e.g. Cl⁻, Br⁻), a pseudohalide (e.g. CN⁻ or OCN⁻), an acetylacetonate or a carboxylate (e.g. acetate).

In an exemplary embodiment of the present invention, the palladium complex of component (i) is of Formula (XI) 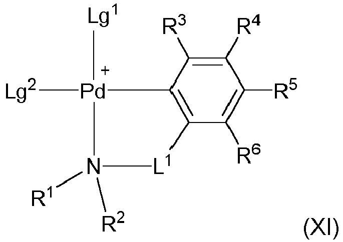 wherein
L¹ is a bivalent linker;
R' and R² are selected independently from each other from hydrogen and C₁₋₄alkyl; or R¹ and R² together with the nitrogen atom to which they are attached form a five- or six-membered ring (which might be an aromatic or non-aromatic ring);
R³, R⁴, R⁵, and R⁶ are selected independently from each other from hydrogen and C₁₋₄alkyl; or at least two of the residues R³ to R⁶ which are adjacent to each other form together with the carbon atoms to which they are attached a five- or six-membered aromatic ring and, if present, the remaining residues (i.e. those of the residues R³ to R⁶ which do not form a ring) are selected independently from each other from hydrogen and C₁₋₄alkyl;
Lg¹ and Lg² are independently from each other a non-ionic ligand.

The compound of Formula (XI) might be associated with one or more charge-compensating anions, e.g. one or more weakly coordinating anions such as [BF₄]⁻, [ClO₄]⁻, [SbF₆]⁻ and [PF₆]⁻. However, other anions might be present as well.

Preferably, ligand Lg² is an N-heterocyclic carbene; and/or ligand Lg¹ is a pyridine, a pyrrole, an imidazole, a pyrazole or a pyrimidine, each of which is optionally substituted with one or more halogens.

According to an exemplary embodiment of Formula (XI),
L¹ is C₁₋₃ alkylene (e.g. -CH₂-; -CH₂-CH₂-; -CH₂-CH₂-CH₂-);
Lg¹ is a pyridine, a pyrrole, an imidazole, a pyrazole or a pyrimidine, each of which is optionally substituted with one or more halogens;
Lg² is an N-heterocyclic carbene.

The N-heterocyclic carbene ligand Lg² can be of Formula (XII) or Formula (XIII): 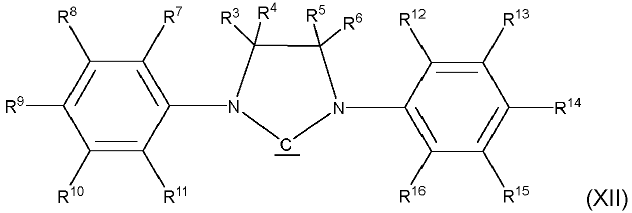
wherein in Formula (XII)
   R³ to R¹⁶ are selected independently from each other from hydrogen and C₁₋₄alkyl; or at least two of the residues R³ to R¹⁶ which are adjacent to each other form together with the carbon atoms to which they are attached a 5- or 6-membered ring and, if present, the remaining residues (i.e. those of the residues R³ to R¹⁶ not forming a ring) are selected independently from each other from hydrogen and C₁₋₄alkyl; 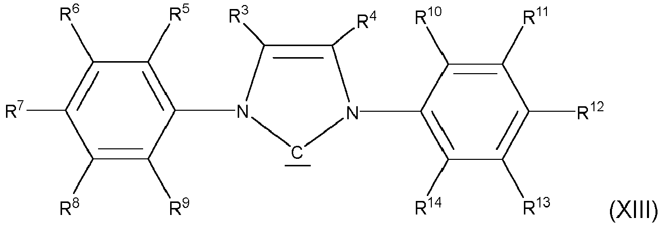
wherein in Formula (XIII)
   R³ to R¹⁴ are selected independently from each other from hydrogen and C₁₋₄ alkyl; or at least two of the residues R³ to R¹⁴ which are adjacent to each other form together with the carbon atoms to which they are attached a 5- or 6-membered ring and, if present, the remaining residues (i.e. those of the residues R³ to R¹⁴ not forming a ring) are selected independently from each other from hydrogen and C₁₋₄alkyl.

An exemplary palladium complex of Formula (XI) has the following Formula (XI-1):

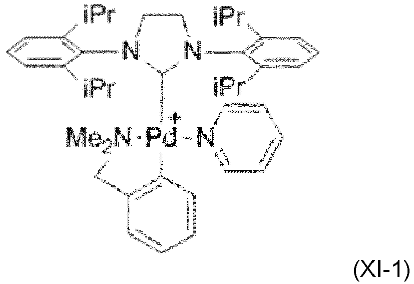

According to an exemplary embodiment of the present invention, the catalyst composition used in the process of the present invention comprises (i) a palladium complex of Formula (XI); and (ii) a polycyclic compound of Formula (Ia). Preferably, ligand Lg² in Formula (XI) is a N-heterocyclic carbene (e.g. of Formula (XII) or (XIII)); and/or ligand Lg¹ is a pyridine, a pyrrole, an imidazole, a pyrazole or a pyrimidine, each of which is optionally substituted with one or more halogens.

According to another exemplary embodiment of the present invention, the catalyst composition used in the process of the present invention comprises (i) a palladium complex of Formula (XI); and (ii) a polycyclic compound of Formula (Ib). Preferably, ligand Lg² in Formula (XI) is a N-heterocyclic carbene (e.g. of Formula (XII) or (XIII)); and/or ligand Lg¹ is a pyridine, a pyrrole, an imidazole, a pyrazole or a pyrimidine, each of which is optionally substituted with one or more halogens.

According to another exemplary embodiment of the present invention, the catalyst composition used in the process of the present invention comprises (i) a palladium complex of Formula (XI); and (ii) a polycyclic compound of Formula (Ic). Preferably, ligand Lg² in Formula (XI) is a N-heterocyclic carbene (e.g. of Formula (XII) or (XIII)); and/or ligand Lg¹ is a pyridine, a pyrrole, an imidazole, a pyrazole or a pyrimidine, each of which is optionally substituted with one or more halogens.

According to another exemplary embodiment of the present invention, the catalyst composition used in the process of the present invention comprises (i) a palladium complex of Formula (XI); and (ii) a polycyclic compound of Formula (Id). Preferably, ligand Lg² in Formula (XI) is a N-heterocyclic carbene (e.g. of Formula (XII) or (XIII)); and/or ligand Lg¹ is a pyridine, a pyrrole, an imidazole, a pyrazole or a pyrimidine, each of which is optionally substituted with one or more halogens.

According to another exemplary embodiment of the present invention, the catalyst composition used in the process of the present invention comprises (i) a palladium complex of Formula (XI); and (ii) a polycyclic compound of Formula (II). Preferably, ligand Lg² in Formula (XI) is a N-heterocyclic carbene (e.g. of Formula (XII) or (XIII)); and/or ligand Lg¹ is a pyridine, a pyrrole, an imidazole, a pyrazole or a pyrimidine, each of which is optionally substituted with one or more halogens.

According to another exemplary embodiment of the present invention, the catalyst composition used in the process of the present invention comprises (i) a palladium complex of Formula (XI); and (ii) a polycyclic compound of Formula (III). Preferably, ligand Lg² in Formula (XI) is a N-heterocyclic carbene (e.g. of Formula (XII) or (XIII)); and/or ligand Lg¹ is a pyridine, a pyrrole, an imidazole, a pyrazole or a pyrimidine, each of which is optionally substituted with one or more halogens.

The palladium complex of component (i) can be of Formula (XIV) 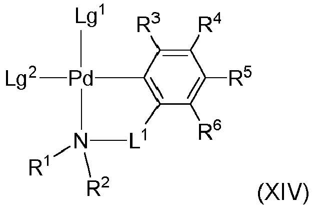 wherein
L¹ is a bivalent linker;
R¹ and R² are selected independently from each other from hydrogen and C₁₋₄ alkyl; or R¹ and R² together with the nitrogen atom to which they are attached form a five- or six-membered ring,
R³, R⁴, R⁵, and R⁶ are selected independently from each other from hydrogen and C₁₋₄ alkyl; or at least two of the residues R³ to R⁶ which are adjacent to each other form together with the carbon atoms to which they are attached a five- or six-membered aromatic ring and, if present, the remaining residues (i.e. those of the residues R³ to R⁶ which do not form a ring) are selected independently from each other from hydrogen and C₁₋₄alkyl,
Lg¹ is a non-ionic ligand,
Lg² is an anionic ligand.

Preferably, the non-ionic ligand Lg¹ in Formula (XIV) is a phosphine.

The anionic ligand Lg² in Formula (XIV) might be an alkyl sulfonate (e.g. mesylate) or an aryl sulfonate (e.g. tosylate). However, other anionic ligands might be used as well.

In an exemplary embodiment of Formula (XIV),
L¹ is phenylene (e.g. 1,2-phenylene) or C₁₋₃alkylene (e.g. -CH₂-; -CH₂-CH₂-; -CH₂-CH₂-CH₂-);
the non-ionic ligand Lg¹ is a phosphine;
the anionic ligand Lg² is an alkyl sulfonate (e.g. mesylate) or an aryl sulfonate (e.g. tosylate).

The phosphine ligand Lg¹ in Formula (XIV) can be phosphine of Formula (XV):

P(R¹)(R²)(R³) (XV)

wherein
R¹ and R² are selected independently from each other from C₁₋₆alkyl, C₅₋₇cycloalkyl (in particular cyclohexyl) and phenyl, wherein phenyl is optionally substituted with one or more C₁₋₄ alkyl;
R³ is biphenyl or binaphthyl, each of which is optionally substituted with one or more R⁴;
R⁴, if present, is C₁₋₆alkyl, -NH₂, -N(H)(C₁₋₆alkyl), -N(C₁₋₆alkyl)₂, -P(Phenyl)₂, or -O-C₁₋₆alkyl.

Exemplary palladium complexes of Formula (XIV) can have one of the following Formulas (XIV-1) to Formula (XIV-7):

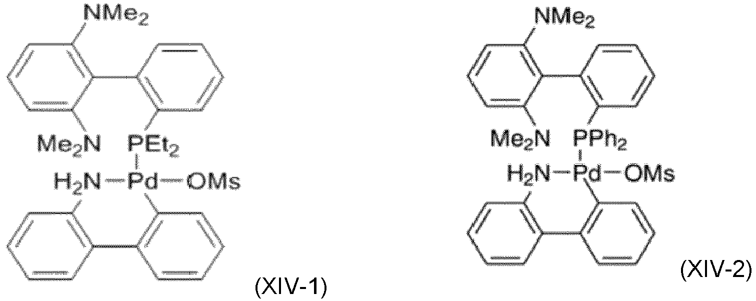

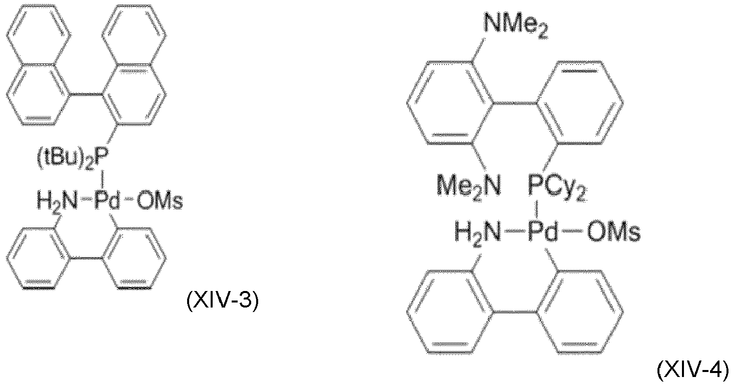

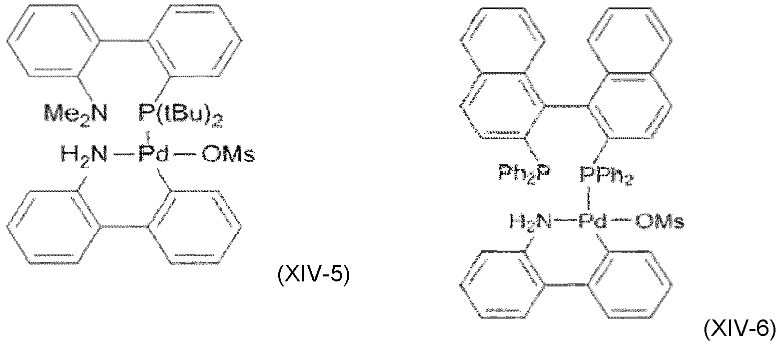

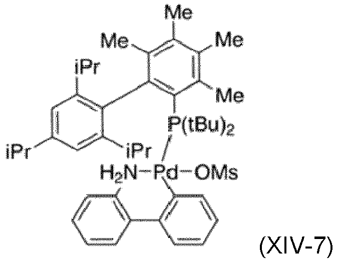

According to another exemplary embodiment, the phosphine ligand Lg¹ in Formula (XIV) is a ferrocenyl diphosphine.

Exemplary palladium complexes of Formula (XIV) containing a ferrocenyl diphosphine ligand are those of Formula (XIV-8) or Formula (XIV-9):

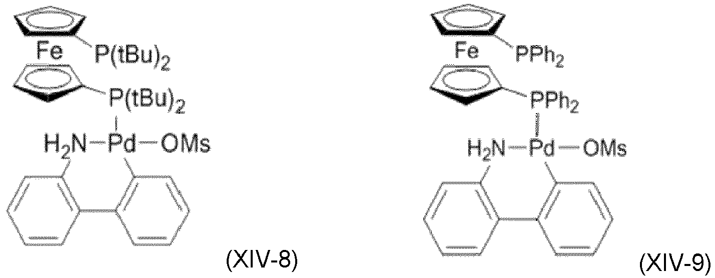

According to another exemplary embodiment, the phosphine ligand Lg¹ in Formula (XIV) is a trialkyl phosphine (e.g. P(C₁₋₆ alkyl)₃). An exemplary palladium complex of Formula (XIV) containing a trialkyl phosphine ligand is the palladium complex of Formula (XIV-10):

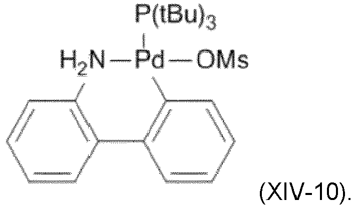

Other exemplary palladium complexes of Formula (XIV) containing a phosphine ligand Lg¹ are those of Formula (XIV-11), (XIV-12), or (XIV-13):

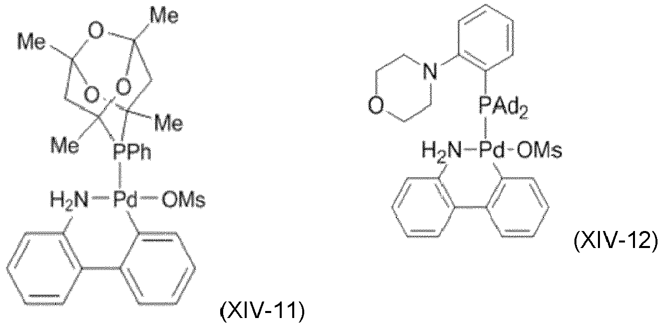

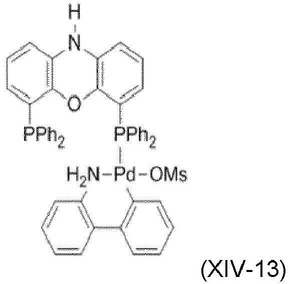

According to an exemplary embodiment of the present invention, the catalyst composition used in the process of the present invention comprises (i) a palladium complex of Formula (XIV); and (ii) a polycyclic compound of Formula (Ia). Preferably, the non-ionic ligand Lg¹ in Formula (XIV) is a phosphine (e.g. a phosphine of Formula (XV), a ferrocenyl diphosphine or a phosphine in one of the Formulas (XIV-11) to (XIV-13)).

According to another exemplary embodiment of the present invention, the catalyst composition used in the process of the present invention comprises (i) a palladium complex of Formula (XIV); and (ii) a polycyclic compound of Formula (Ib). Preferably, the non-ionic ligand Lg¹ in Formula (XIV) is a phosphine (e.g. a phosphine of Formula (XV), a ferrocenyl diphosphine or a phosphine in one of the Formulas (XIV-11) to (XIV-13)).

According to another exemplary embodiment of the present invention, the catalyst composition used in the process of the present invention comprises (i) a palladium complex of Formula (XIV); and (ii) a polycyclic compound of Formula (Ic). Preferably, the non-ionic ligand Lg¹ in Formula (XIV) is a phosphine (e.g. a phosphine of Formula (XV), a ferrocenyl diphosphine or a phosphine in one of the Formulas (XIV-11) to (XIV-13)).

According to another exemplary embodiment of the present invention, the catalyst composition used in the process of the present invention comprises (i) a palladium complex of Formula (XIV); and (ii) a polycyclic compound of Formula (Id). Preferably, the non-ionic ligand Lg¹ in Formula (XIV) is a phosphine (e.g. a phosphine of Formula (XV), a ferrocenyl diphosphine or a phosphine in one of the Formulas (XIV-11) to (XIV-13)).

According to another exemplary embodiment of the present invention, the catalyst composition used in the process of the present invention comprises (i) a palladium complex of Formula (XIV); and (ii) a polycyclic compound of Formula (II). Preferably, the non-ionic ligand Lg¹ in Formula (XIV) is a phosphine (e.g. a phosphine of Formula (XV), a ferrocenyl diphosphine or a phosphine in one of the Formulas (XIV-11) to (XIV-13)).

According to an exemplary embodiment of the present invention, the catalyst composition used in the process of the present invention comprises (i) a palladium complex of Formula (XIV); and (ii) a polycyclic compound of Formula (III). Preferably, the non-ionic ligand Lg¹ in Formula (XIV) is a phosphine (e.g. a phosphine of Formula (XV), a ferrocenyl diphosphine or a phosphine in one of the Formulas (XIV-11) to (XIV-13)).

The palladium complex of component (i) can be of Formula (XVI):

Pd(L)₃₋₄ (XVI)

wherein
the ligands L are independently from each other a triaryl phosphine (preferably a triphenyl phosphine), wherein each aryl group (which preferably is a phenyl group) is optionally substituted with one or more R¹;
R¹, if present, is C₁₋₄haloalkyl (e.g. -CF₃), C₁₋₄alkyl (e.g. methyl) or C₁₋₄alkoxy (e.g. methoxy).

Exemplary palladium complexes of Formula (XVI) are those of Formula (XVI-1) or (XVI-2):

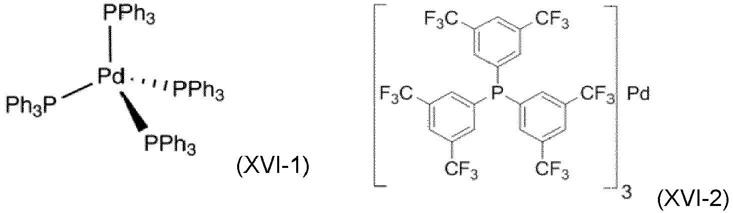

According to an exemplary embodiment of the present invention, the catalyst composition used in the process of the present invention comprises (i) a palladium complex of Formula (XVI); and (ii) a polycyclic compound of Formula (Ia).

According to another exemplary embodiment of the present invention, the catalyst composition used in the process of the present invention comprises (i) a palladium complex of Formula (XVI); and (ii) a polycyclic compound of Formula (Ib).

According to another exemplary embodiment of the present invention, the catalyst composition used in the process of the present invention comprises (i) a palladium complex of Formula (XVI); and (ii) a polycyclic compound of Formula (Ic).

According to another exemplary embodiment of the present invention, the catalyst composition used in the process of the present invention comprises (i) a palladium complex of Formula (XVI); and (ii) a polycyclic compound of Formula (Id).

According to another exemplary embodiment of the present invention, the catalyst composition used in the process of the present invention comprises (i) a palladium complex of Formula (XVI); and (ii) a polycyclic compound of Formula (II).

According to another exemplary embodiment of the present invention, the catalyst composition used in the process of the present invention comprises (i) a palladium complex of Formula (XVI); and (ii) a polycyclic compound of Formula (III).

The palladium complex of component (i) can be of Formula (XVII): 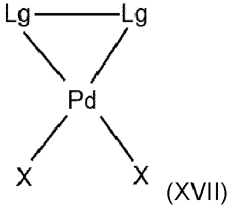 wherein
Lg-Lg is a chelating ferrocenyl diphosphine ligand,
X is a halide (e.g. CI).

Exemplary palladium complexes of Formula (XVII) are those of Formula (XVII-1) or (XVII-2):

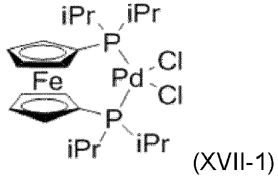

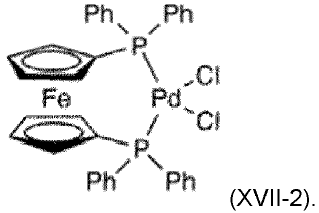

According to an exemplary embodiment of the present invention, the catalyst composition used in the process of the present invention comprises (i) a palladium complex of Formula (XVII); and (ii) a polycyclic compound of Formula (Ia).

According to another exemplary embodiment of the present invention, the catalyst composition used in the process of the present invention comprises (i) a palladium complex of Formula (XVII); and (ii) a polycyclic compound of Formula (Ib).

According to another exemplary embodiment of the present invention, the catalyst composition used in the process of the present invention comprises (i) a palladium complex of Formula (XVII); and (ii) a polycyclic compound of Formula (Ic).

According to another exemplary embodiment of the present invention, the catalyst composition used in the process of the present invention comprises (i) a palladium complex of Formula (XVII); and (ii) a polycyclic compound of Formula (Id).

According to another exemplary embodiment of the present invention, the catalyst composition used in the process of the present invention comprises (i) a palladium complex of Formula (XVII); and (ii) a polycyclic compound of Formula (II).

According to another exemplary embodiment of the present invention, the catalyst composition used in the process of the present invention comprises (i) a palladium complex of Formula (XVII); and (ii) a polycyclic compound of Formula (III).

It might be preferred that no compound comprising a transition metal other than palladium is present in the catalyst composition.

The molar ratio between the palladium compound (component (i)) and the one or more polycyclic compounds of component (ii) might be in the range of from 100/1 to 1/100, more preferably 10/1 to 1/10.

As already outlined above, the process based on an intermolecular decarboxylative carbon-carbon cross-coupling reaction comprises
- mixing a first reactant, a second reactant and the catalyst composition described above, thereby providing a reaction medium, wherein the first reactant is a compound which comprises a heterocyclic aromatic ring AR1 to which a carboxylic acid group, a carboxylate group or a carboxylic acid ester group is directly attached, and the second reactant is a compound which comprises an aromatic ring AR2 to which a halogen atom (e.g. Cl, Br or I), a pseudohalogen group (e.g. cyano, cyanate or isocyanate) or a sulfonate group (e.g. trifluoromethanesulfonate group -O-Tf) is directly attached,
- irradiating the reaction medium by an external light source, thereby coupling the heterocyclic aromatic ring AR1 of the first reactant to the aromatic ring AR2 of the second reactant by a decarboxylative carbon-carbon cross-coupling reaction.

The product obtained by the process is a heteroaromatic biaryl compound, i.e. a compound which comprises a heterocyclic aromatic ring AR1 and an aromatic ring AR2 (which might be a carbocyclic or heterocyclic aromatic ring) being linked to each other by a carbon-carbon single bond. The heterocyclic aromatic ring AR1 originates from the first reactant, and the aromatic ring AR2 originates from the second reactant.

Preferably, the heterocyclic aromatic ring AR1 of the first reactant is 5- or 6-membered aromatic ring which contains one or more heteroatoms (e.g. up to three heteroatoms) being selected from nitrogen, oxygen and sulfur. The carboxylic acid group, carboxylate group or carboxylic acid ester group is attached to a carbon ring atom of the heterocyclic aromatic ring. Optionally, a further 5- or 6-membered aromatic ring might be fused to the heterocyclic aromatic ring, thereby forming a polycyclic aromatic ring system.

Exemplary first reactants include a substituted or unsubstituted pyridine carboxylic acid or a carboxylate or an ester thereof, a substituted or unsubstituted benzopyridine carboxylic acid or a carboxylate or an ester thereof, a pyridazine carboxylic acid or a carboxylate or an ester thereof, a benzopyridazine carboxylic acid or a carboxylate or an ester thereof, a pyrimidine carboxylic acid or a carboxylate or an ester thereof, a benzopyrimidine carboxylic acid or a carboxylate or an ester thereof, a pyrazine carboxylic acid or a carboxylate or an ester thereof, a benzopyrazine carboxylic acid or a carboxylate or an ester thereof, a pyrrole carboxylic acid or a carboxylate or an ester thereof, a benzopyrrole carboxylic acid or a carboxylate or an ester thereof, an imidazole carboxylic acid or a carboxylate or an ester thereof, a benzimidazole carboxylic acid or a carboxylate or an ester thereof, an oxazole carboxylic acid or a carboxylate or an ester thereof, a benzoxazole carboxylic acid or a carboxylate or an ester thereof, an isoxazole carboxylic acid or a carboxylate or an ester thereof, a benzisoxazole carboxylic acid or a carboxylate or an ester thereof, a thiazole carboxylic acid or a carboxylate or an ester thereof, a benzothiazole carboxylic acid or a carboxylate or an ester thereof, an isothiazole carboxylic acid or a carboxylate or an ester thereof, and a benzisothiazole carboxylic acid or a carboxylate or an ester thereof.
A carboxylic acid group is represented by the following formula: -COOH
A carboxylate group is a deprotonated carboxylic acid group and is therefore represented by the following formula: -COO⁻

If a carboxylic acid ester group is attached to the heterocyclic aromatic ring of the first reactant, it may have the following formula (XVIII):

-C(O)O-A (XVIII)

wherein the residue A is derived from a N-hydroxy compound.

Accordingly, the carboxylic acid ester group might be obtained by reacting a carboxylic acid group with a N-hydroxy compound.

Exemplary N-hydroxy compounds from which residue A of the carboxylic acid ester group might be derived include oximes (ketoximes or aldoximes), N-hydroxy phthalimides, N-hydroxypyridine-2-thiones, and N-hydroxy benzotriazoles.

According to an exemplary embodiment, the residue A in Formula (XVIII) might have one of the following Formulas (XIX-1) to (XIX-6): 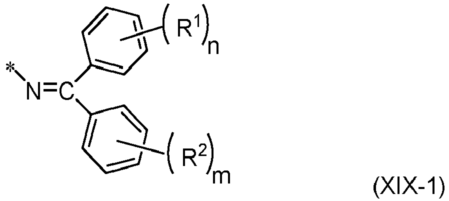 wherein in Formula (XIX-1)
n is 0 or 1,
m is 0 or 1,
R' and R² are selected independently from each other from halogen (e.g. Cl or F) and halo-C₁₋₄alkyl (e.g. CF₃); 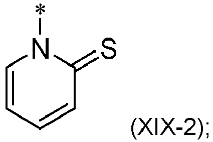 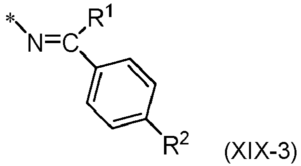
wherein in Formula (XIX-3)
R¹ is hydrogen or CF₃,
R² is hydrogen or fluoro; 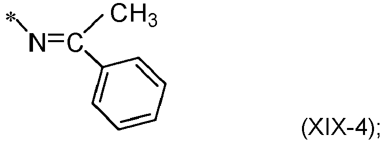 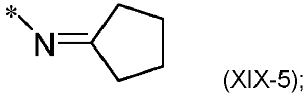 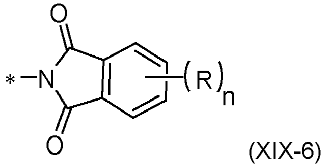
wherein in Formula (XIX-6)
n is an integer from 0 to 4,
R is halogen (e.g. CI).

In Formulas (XIX-1) to (XIX-6), ∗- denotes the bond by which residue A is connected to the carboxylic acid ester group.

If n is 0 in Formula (XIX-1) or (XIX-6) or m is 0 in Formula (XIX-1), the aromatic ring is unsubstituted.

It might be preferred that the first reactant does not comprise a boron-carbon bond, a tin-carbon bond, a zinc-carbon bond or a magnesium-carbon bond.

The aromatic ring AR2 of the second reactant is preferably a 5- or 6-membered aromatic ring. It can be a carbocyclic ring (i.e. a ring made of carbon atoms only) or heterocyclic aromatic ring. Optionally, it is fused to a further 5- or 6-membered aromatic ring, thereby forming a polycyclic aromatic ring system.

Attached to the aromatic ring AR2 of the second reactant is a halogen atom (e.g. Cl, Br or I), a pseudohalogen group (e.g. cyano, cyanate or isocyanate) or a sulfonate group (such as a trifluoromethanesulfonate group -O-Tf).

If the aromatic ring AR2 is a heterocyclic aromatic ring, it is preferably a 5- or 6-membered aromatic ring which contains one or more (e.g. up to three) heteroatoms being selected from nitrogen, oxygen and sulfur. The halogen atom, the pseudohalogen group or the sulfonate group is attached to a carbon ring atom of the heterocyclic aromatic ring.

It might be preferred that neither the first reactant nor the second reactant comprises a boron-carbon bond, a tin-carbon bond, a zinc-carbon bond or a magnesium-carbon bond.

During the cross-coupling reaction, the carboxylic acid group, the carboxylate group or the carboxylic acid ester group is split off from the heterocyclic aromatic ring AR1; and the halogen atom, the pseudohalogen group or the sulfonate group is split off from the aromatic ring AR2; and the aromatic rings AR1 and AR2 are coupled to each other via a single bond between the carbon ring atom of AR1 to which the carboxylic acid group, the carboxylate group or the carboxylic acid ester group was attached and the carbon ring atom of AR2 to which the halogen atom, the pseudohalogen group or the sulfonate group was attached.

The reaction outlined above represents an intermolecular decarboxylative cross-coupling reaction between a first reactant and a second reactant. Typically, the first reactant and the second reactant differ from each other (i.e. they are compounds which differ in chemical structure).

Alternatively, the heterocyclic aromatic ring AR1 and the carbocyclic or heterocyclic aromatic ring AR2 might be coupled to each other by an intramolecular decarboxylative cross-coupling reaction (i.e. each of the reactant molecules containing both the heterocyclic aromatic ring AR1 and the carbocyclic or heterocyclic aromatic ring AR2).

The process based on an intramolecular decarboxylative carbon-carbon cross-coupling reaction comprises
- mixing a reactant and the catalyst composition described above, thereby providing a reaction medium, wherein the reactant is a compound which comprises a heterocyclic aromatic ring AR1 to which a carboxylic acid group, a carboxylate group or a carboxylic acid ester group is attached and an aromatic ring AR2 to which a halogen atom (e.g. Cl, Br or I), a pseudohalogen group (e.g. cyano, cyanate or isocyanate) or a sulfonate group (e.g. trifluoromethanesulfonate group -O-Tf) is attached,
- irradiating the reaction medium by an external light source, thereby coupling the heterocyclic aromatic ring AR1 and the aromatic ring AR2 of the reactant to each other by a decarboxylative carbon-carbon cross-coupling reaction.

The aromatic ring AR2 can be a carbocyclic aromatic ring or a heterocyclic aromatic ring.

The preferred heterocyclic aromatic ring AR1 and (carbocyclic or heterocyclic) aromatic ring AR2 of the intramolecular coupling reaction correspond to those of the intermolecular coupling reaction. However, for the intramolecular coupling reaction, each of the reactant molecules contains both the aromatic ring AR1 and the aromatic ring AR2 linked to each other by a bivalent linker unit L.

It might be preferred that the reactant does not comprise a boron-carbon bond, a tin-carbon bond, a zinc-carbon bond or a magnesium-carbon bond.

The following statements apply to both the intermolecular and the intramolecular decarboxylative carbon-carbon cross-coupling reactions.

Typically, the reactant(s) and the catalyst composition are mixed with each other in a solvent. The solvent may comprise a polar aprotic solvent. The solvent may comprise a high-boiling liquid having a boiling point of at least 120°C. Exemplary polar aprotic solvents include dimethylsulfoxide (DMSO), dimethylformamide (DMF), dimethylacetamide, sulfolane and ethylacetate. The reactant(s) and the components of the catalyst composition (i.e. components (i) to (ii) and optionally component (iii)) can be added to the solvent in any order. Just as an example, each of the components of the catalyst composition might be added separately to the solvent, or the components of the catalyst composition are pre-mixed and the pre-mixture is subsequently added to the solvent.

Typically, the external light source is a source which generates artificial light.

The external light source might be a lamp such as an UV or UV/VIS lamp, a LED, a laser, or any combination of two or more of these external light sources.

The light emitted by the external light source may have a wavelength in the range of from 254 nm to 800 nm, preferably 360 nm to 550 nm. The light can be a monochromatic light; or may cover a continuous wavelength spectrum (e.g. in the UV and/or visible spectrum). The light reaching the reaction medium may have an irradiance of 10 to 2000 milliwatts per square centimeter. However, lower or higher irradiance values can be used as well.

Optionally, the reaction medium might be heated to a temperature of 40°C to 90°C, more preferably 50°C to 80°C, for assisting the decarboxylative carbon-carbon cross-coupling reaction. However, lower or higher reaction temperatures might be used as well.

It might be preferred that no compound comprising a transition metal other than palladium is present in the reaction medium.

Furthermore, the present invention relates to the use of the catalyst composition described above as a catalyst for coupling a heterocyclic aromatic ring AR1 and a carbocyclic or heterocyclic aromatic ring AR2 to each other by a light-assisted decarboxylative carbon-carbon cross-coupling reaction. As already mentioned above, the cross-coupling reaction might be an intermolecular cross-coupling or an intramolecular cross-coupling. The preferred use is for synthesis of biaryl compounds. As known to the skilled person, *"light assisted"* means that the reaction medium in which the decarboxylative cross-coupling reaction is carried out is irradiated by an external light source (e.g. lamps such as UV or UV/VIS lamps, LEDs, laser, etc.).

### Examples

### Inventive Example 1 (IE1)

In Inventive Example 1 (IE1), the following reactants were used:
- Picolinic acid ester (First Reactant), i.e. a compound which comprises a heterocyclic aromatic ring AR1 to which a carboxylic acid ester group is attached; and
- 4-bromobenzotrifluoride (Second Reactant), i.e. a compound which comprises an aromatic ring AR2 to which a halogen atom is attached.

The picolinic acid ester had the following chemical formula:

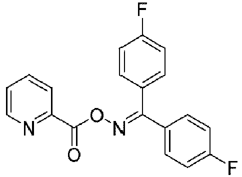

The catalyst composition used in Inventive Example 1 contained (i) palladium acetylacetonate (i.e. a palladium compound), (ii) a polycyclic compound of Formula (I-1) outlined above, and (iii) tBuXPhos which is a commercially available phosphine of Formula (IV-4) outlined above.

The solvent in which the first and second reactants and the components of the catalyst composition were mixed was ethylacetate.

The reaction medium was irradiated with a blue light emitting diode.

The reactants and the desired reaction product to be obtained via decarboxylative cross-coupling are illustrated by the following reaction scheme:

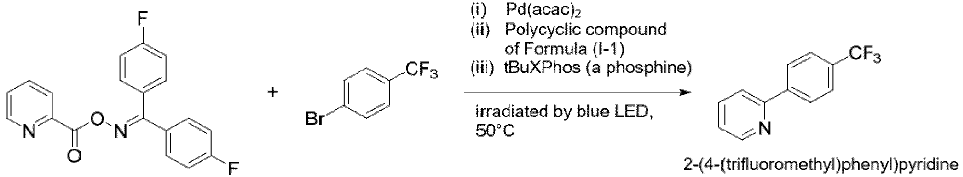

Details of the preparation process are as follows:
To a ½ dram vial containing a stir bar, 150 µL of 0.005 M stock solution of the polycyclic compound of Formula (I-1) in CH₃CN was introduced; the solvent was then evaporated in a vacuum oven leaving behind the polycyclic compound of Formula (I-1). 38.1 mg of the picolinic acid ester and 1.1 mg of Pd(acac)₂ were then added to the vial. The vial was transferred to a glovebox containing a nitrogen atmosphere. In the glovebox, degassed reagents of 10.5 µL of 4-bromobenzotrifluoride, 75 µL of 0.05 M stock solution of t-BuXPhos in dioxane, and 675 µL of ethylacetate solvent were added to the vial. The vial was tightly capped, sealed with parafilm, and removed from the glovebox. Upon exiting the glovebox, an electrical tape was wrapped around the cap of the vial to ensure that oxygen contamination did not occur. The vial was then placed in the Hepatochem photoreactor setup, where temperature (at 50°C) was controlled by a recirculating coolant (propylene glycol/water=1/1, v/v). The vial was illuminated with a Kessil Tuna Blue LED light (A160WE).

After 16 hours, the reaction vial was allowed to cool to room temperature before being opened. 50 µL of 1.6 M biphenyl in ethylacetate (internal standard for HPLC analysis) was added to the vial. For HPLC analysis, 75 µL of the mixture was taken from the vial, filtered through a silica column, and washed with 1425 µL of CH₃CN; all solution was collected for HPLC analysis. For ¹⁹F-NMR analysis, 20 µL of the mixture was taken from the vial and added 600 µL of CDCl₃ (containing 0.1% v/v fluorobenzene as internal standard).

¹⁹F-NMR analysis confirmed that the desired decarboxylative cross-coupling reaction product 2-(4-(trifluoromethyl)phenyl)pyridine product was formed.

### Comparative Example 1a (CE1a)

In Comparative Example 1a (CE1a), the same procedure as in Inventive Example 1 was applied, except that the reaction medium was not irradiated by the LED.

¹⁹F-NMR analysis confirmed that the desired decarboxylative cross-coupling reaction product 2-(4-(trifluoromethyl)phenyl)pyridine product was not formed.

### Comparative Example 1b (CE1b)

In Comparative Example 1b (CE1b), the same procedure as in Inventive Example 1 was applied, except that the compound of component (ii), i.e. the polycyclic compound of Formula (I-1), was not added to the reaction medium.

¹⁹F-NMR analysis confirmed that the desired decarboxylative cross-coupling reaction product 2-(4-(trifluoromethyl)phenyl)pyridine product was not formed.

The results of Inventive Example 1 and Comparative Examples 1a and 1b are summarized in Table 1.

**Table 1: Results of IE1, CE1a and CE1b**

| | **Reactants** | **Catalyst Composition** | **Irradation by LED?** | **Desired reaction product formed?** |
|---|---|---|---|---|
| **IE1** | Picolinic acid ester and 4-bromobenzotrifluoride | (i) Pd(acac)₂ | Yes | Yes |
| | | (ii) polycyclic compound of Formula (I-1) | | |
| | | (iii) a phosphine (tBuXPhos) | | |
| **CE1a** | Picolinic acid ester and 4-bromobenzotrifluoride | (i) Pd(acac)₂ | No | No |
| | | (ii) polycyclic compound of Formula (I-1) | | |
| | | (iii) a phosphine (tBuXPhos) | | |
| **CE1b** | Picolinic acid ester and 4-bromobenzotrifluoride | (i) Pd(acac)₂ | Yes | No |
| | | (iii) a phosphine (tBuXPhos) | | |

From the Inventive Example 1 and the Comparative Examples 1a and 1b, the following can be concluded:
If a heteroaryl carboxylic acid or a carboxylate or an ester thereof is reacted with a (hetero)aryl halide (or a (hetero)aryl pseudohalide or a (hetero)aryl sulfonate) in a reaction medium
- to which the catalyst composition of the present invention has been added and
- which is irradiated by an external light source,
a heteroaromatic biaryl compound can be formed by a decarboxylative carbon-carbon cross-coupling reaction even at rather moderate reaction temperature (e.g. 50°C).

However, no heteroaromatic biaryl compound is formed from these reactants at moderate reaction temperature if
- the reaction medium comprises the palladium compound and is irradiated by an external light source but no compound of component (ii) is present, or
- the reaction medium comprises both the palladium compound and at least one compound of component (ii) but is not irradiated by an external light source.

While the invention has been described in detail and with reference to specific embodiments thereof, it will be apparent to one skilled in the art that various changes and modifications can be made therein without departing from the spirit and scope thereof. Thus, it is intended that the invention covers the modifications and variations of this invention provided they come within the scope of the appended claims and their equivalents.

As described above, the present invention relates to the following embodiments {01} to {57}:
Embodiment {01} relates to a process which comprises
   - mixing a first reactant, a second reactant and a catalyst composition, thereby providing a reaction medium, wherein
      the first reactant is a compound which comprises a heterocyclic aromatic ring AR1 to which a carboxylic acid group, a carboxylate group or a carboxylic acid ester group is attached,
      the second reactant is a compound which comprises an aromatic ring AR2 to which a halogen atom (e.g. Cl, Br or I), a pseudohalogen group (e.g. cyano, cyanate or isocyanate) or a sulfonate group (such as a trifluoromethanesulfonate group -O-Tf) is attached,
      the catalyst composition comprises
         (i) a palladium compound which is selected from a palladium salt or a palladium complex or a mixture thereof,
         (ii) a polycyclic compound of Formula (I), (II) or (III): 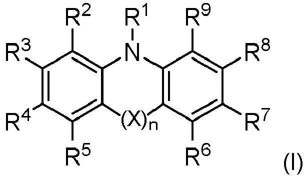 wherein in Formula (I)
            n is 0 or 1,
            X is O, C(R¹⁰)(R¹¹), or N(R¹²),
            R' to R¹² are selected independently from each other from hydrogen, aryl and C₁₋₁₀alkyl, wherein aryl and C₁₋₁₀alkyl are optionally substituted with one or more substituents R¹³, wherein the one or more substituents R¹³, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 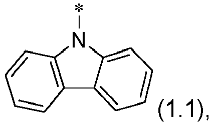 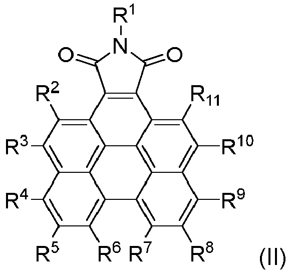
            wherein in Formula (II)
            R¹ is selected from C₁₋₁₆alkyl and aryl, each of which is optionally substituted with one or more substituents R¹², and/or C₁₋₁₆alkyl is optionally interrupted by one or more oxygen atoms;
            R² to R¹¹ are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms; or at least two of the residues R² to R¹¹ which are adjacent to each other form together with the carbon atoms to which they are attached a five- or six-membered aromatic ring and, if present, the remaining residues (i.e. those of the residues R² to R¹¹ which do not form a ring) are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms;
            wherein the one or more substituents R¹², if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 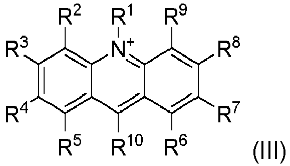
            wherein in Formula (III)
            R¹ is selected from C₁₋₁₆alkyl and aryl, each of which is optionally substituted with one or more substituents R¹¹, and/or C₁₋₁₆alkyl is optionally interrupted by one or more oxygen atoms;
            R² to R⁹ are selected independently from each other from hydrogen, C₁₋₁₆alkyl and aryl, wherein C₁₋₁₆alkyl and aryl are optionally substituted with one or more substituents R¹¹, and/or C₁₋₁₆alkyl is optionally interrupted by one or more oxygen atoms;
            R¹⁰ is aryl which is optionally substituted with one or more substituents R¹²;
            wherein the one or more substituents R¹¹, if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl);
            wherein the one or more substituents R¹², if present, are selected independently from each other from C₁₋₆alkyl and aryl (e.g. phenyl), wherein aryl is optionally substituted with one or more C₁₋₆alkyl;
   - irradiating the reaction medium by an external light source, thereby coupling the heterocyclic aromatic ring AR1 of the first reactant to the aromatic ring AR2 of the second reactant by a decarboxylative carbon-carbon cross-coupling reaction.
Embodiment {02} relates to a process which comprises
   - mixing a reactant and a catalyst composition, thereby providing a reaction medium, wherein
      the reactant is a compound which comprises a heterocyclic aromatic ring AR1 to which a carboxylic acid group, a carboxylate group or a carboxylic acid ester group is attached and an aromatic ring AR2 to which a halogen atom (Cl, Br or I), a pseudohalogen group (e.g. cyano, cyanate or isocyanate) or a sulfonate group (e.g. trifluoromethanesulfonate group -O-Tf) is attached,
      the catalyst composition comprises
         (i) a palladium compound which is selected from a palladium salt or a palladium complex or a mixture thereof,
         (ii) a polycyclic compound of Formula (I), (II) or (III): 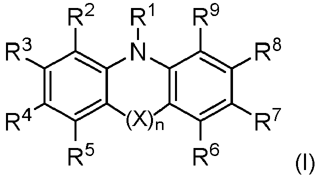 wherein in Formula (I)
            n is 0 or 1,
            X is O, C(R¹⁰)(R¹¹), or N(R¹²),
            R¹ to R¹² are selected independently from each other from hydrogen, aryl and C₁₋₁₀alkyl, wherein aryl and C₁₋₁₀alkyl are optionally substituted with one or more substituents R¹³, wherein the one or more substituents R¹³, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 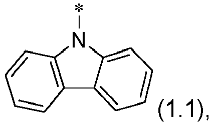 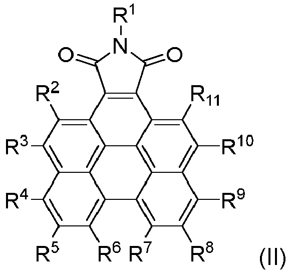
            wherein in Formula (II)
            R¹ is selected from C₁₋₁₆alkyl and aryl, each of which is optionally substituted with one or more substituents R¹², and/or C₁₋₁₆alkyl is optionally interrupted by one or more oxygen atoms;
            R² to R¹¹ are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms; or at least two of the residues R² to R¹¹ which are adjacent to each other form together with the carbon atoms to which they are attached a five- or six-membered aromatic ring and, if present, the remaining residues (i.e. those of the residues R² to R¹¹ which do not form a ring) are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms;
            wherein the one or more substituents R¹², if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 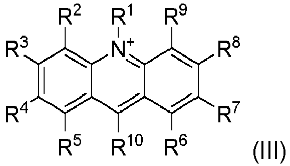
            wherein in Formula (III)
            R¹ is selected from C₁₋₁₆alkyl and aryl, each of which is optionally substituted with one or more substituents R¹¹, and/or C₁₋₁₆alkyl is optionally interrupted by one or more oxygen atoms;
            R² to R⁹ are selected independently from each other from hydrogen, C₁₋₁₆alkyl and aryl, wherein C₁₋₁₆alkyl and aryl are optionally substituted with one or more substituents R¹¹, and/or C₁₋₁₆alkyl is optionally interrupted by one or more oxygen atoms;
            R¹⁰ is aryl which is optionally substituted with one or more substituents R¹²;
            wherein the one or more substituents R¹¹, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl);
            wherein the one or more substituents R¹², if present, are selected independently from each other from C₁₋₆alkyl and aryl (e.g. phenyl), wherein aryl is optionally substituted with one or more C₁₋₆alkyl;
   - irradiating the reaction medium by an external light source, thereby coupling the heterocyclic aromatic ring AR1 and the aromatic ring AR2 of the reactant to each other by a decarboxylative carbon-carbon cross-coupling reaction.
Embodiment {03} relates to the process according to embodiment {01} or embodiment {02}, wherein R¹ in Formula (I) is selected from phenyl, naphthyl and biphenyl, each of which is optionally substituted with one or more substituents R¹³, wherein R¹³ has the same meaning as in embodiment {01} or embodiment {02}.
Embodiment {04} relates to the process according to one of the embodiments {01} to {03}, wherein in Formula (I)
   n is 0;
   R¹ is selected from naphthyl (e.g. 1-naphthyl or 2-naphthyl), phenyl and biphenyl, each of which is optionally substituted with one or more R¹³;
   R² to R⁹ are selected independently from each other from hydrogen, C₁₋₆alkyl, phenyl, biphenyl and naphthyl, wherein phenyl, biphenyl and naphthyl are optionally substituted with one or more substituents R¹³;
   wherein, if present, the one or more substituents R¹³, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 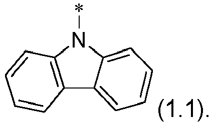
Embodiment {05} relates to the process according to embodiment {04}, wherein the polycyclic compound is a compound of Formula (I-1): 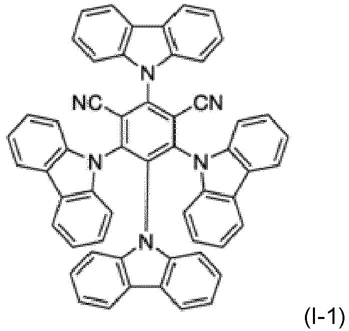
Embodiment {06} relates to the process according to one of the embodiments {01} to {03}, wherein in Formula (I)
   n is 1;
   X is O;
   R¹ is naphthyl (e.g. 1-naphthyl) which is optionally substituted with one or more substituents R¹³; R², R³, R⁵, R⁶, R⁸ and R⁹ are selected independently from each other from hydrogen and C₁₋₆alkyl;
   R⁴ and R⁷ are selected independently from each other from phenyl, biphenyl and naphthyl, each of which is optionally substituted with one or more substituents R¹³;
   wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).
Embodiment {07} relates to the process according to embodiment {06}, wherein the polycyclic compound is a compound of Formula (I-2), (I-3), (I-4) or (I-5): 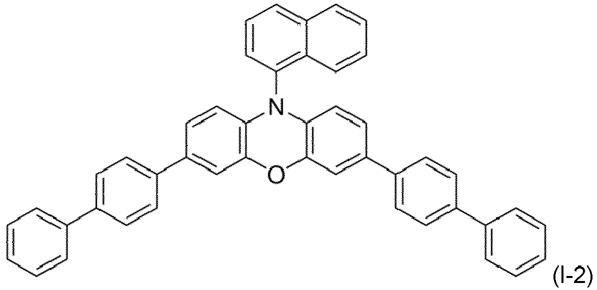 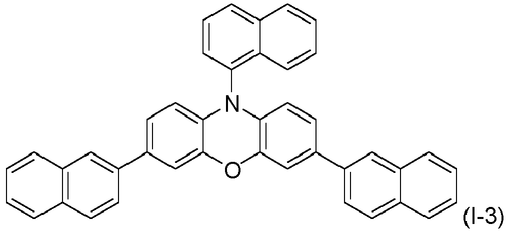 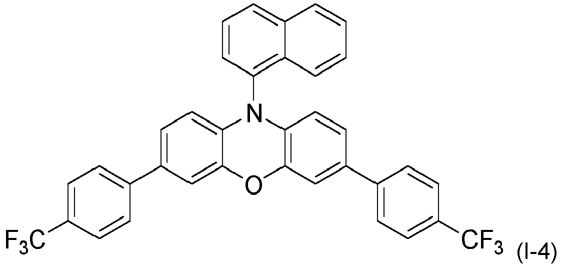 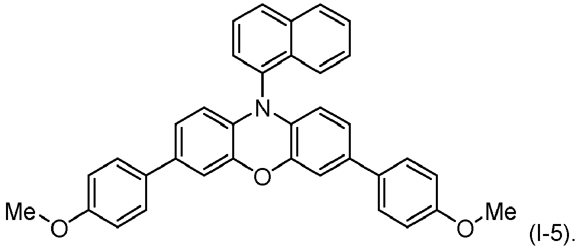
Embodiment {08} relates to the process according to one of the embodiments {01} to {03},
   wherein in Formula (I)
   n is 1;
   X is C(R¹⁰)(R¹¹), wherein R¹⁰ and R¹¹ are selected independently from each other from hydrogen and C₁₋₆alkyl;
   R¹ is selected from naphthyl (e.g. 1-naphthyl or 2-naphthyl), phenyl and biphenyl, each of which is optionally substituted with one or more substituents R¹³;
   R², R³, R⁵, R⁶, R⁸ and R⁹ are selected independently from each other from hydrogen and C₁₋₆alkyl;
   R⁴ and R⁷ are selected independently from each other from phenyl, naphthyl and biphenyl, each of which is optionally substituted with one or more substituents R¹³; wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl) and -S(O)₂(phenyl).
Embodiment {09} relates to the process according to embodiment {08}, wherein the polycyclic compound is a compound of one of the Formulas (I-6.1) to (I-6.7): 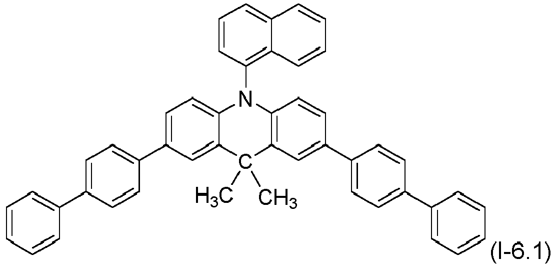 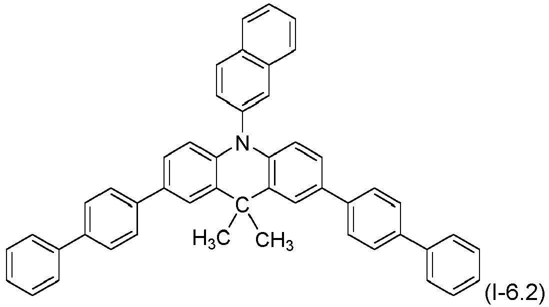 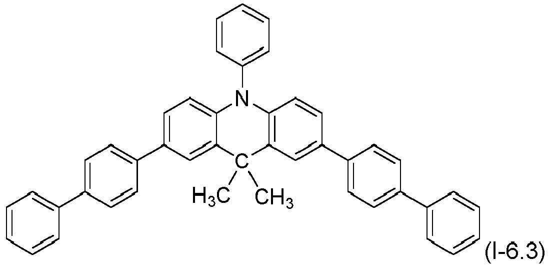 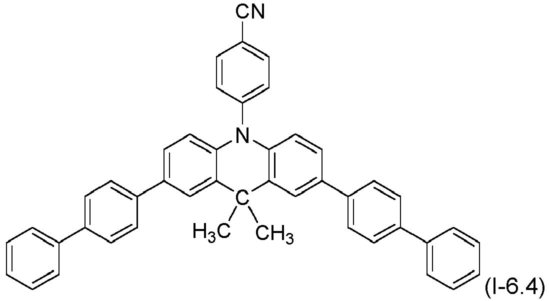 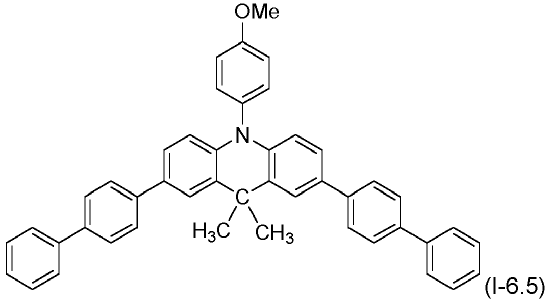 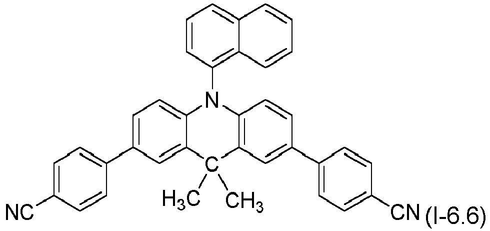 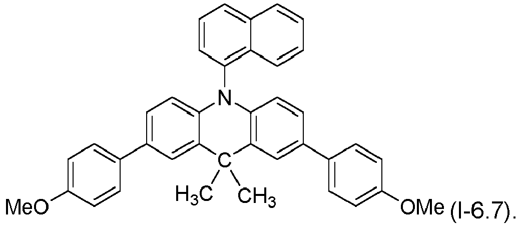
Embodiment {10} relates to the process according to one of the embodiments {01} to {03},
   wherein in Formula (I)
   n is 1;
   Xis N(R¹²);
   R¹ and R¹² are selected independently from each other from phenyl, naphthyl (such as 2-naphthyl or 1-naphthyl) and biphenyl, each of which is optionally substituted with one or more substituents R¹³;
   R² to R⁹ are selected independently from each other from hydrogen and C₁₋₆alkyl; or at least two, preferably all of the residues R³, R⁴, R⁷, and R⁸ are a phenyl group which is optionally substituted with one or more substituents R¹³ and the residues R², R⁵, R⁶ and R⁹ are selected independently from each other from hydrogen and C₁₋₆alkyl;
   wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).
Embodiment {11} relates to the process according to embodiment {10}, wherein the polycyclic compound is a compound of Formula (I-7) or (I-8): 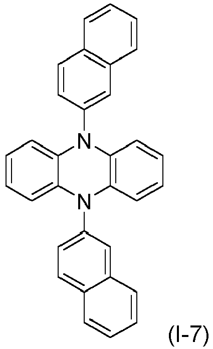 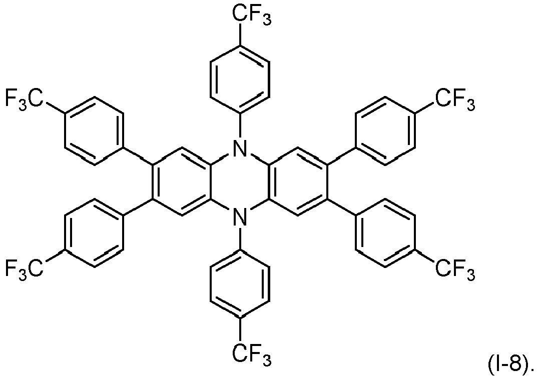
Embodiment {12} relates to the process according to one of the embodiments {01} to {03},
   wherein in Formula (II)
   R¹ is C₁₋₁₂alkyl which is optionally substituted with one or more substituents being selected from -OH, halogen (e.g. F, CI), -NH₂, -NH(C₁₋₆alkyl) and -N(C₁₋₆alkyl)₂;
   R⁴, R⁷, and R⁹ are selected independently from each other from hydrogen, aryl (e.g. phenyl), heteroaryl, C₁₋₆alkyl and -O-(C₁₋₆alkyl), wherein aryl and heteroaryl are optionally substituted with one or more substituents which are selected independently from each other from C₁₋₆alkyl, C₁₋₆haloalkyl (e.g. C₁₋₆fluoroalkyl) and -O-(C₁₋₆alkyl);
   R², R³, R⁵, R⁶, R⁸, R¹⁰, and R¹¹ are selected independently from each other from hydrogen and C₁₋₆alkyl.
Embodiment {13} relates to the process according to embodiment {12}, wherein the polycyclic compound is a compound of Formula (II-1), (II-2) or (II-3): 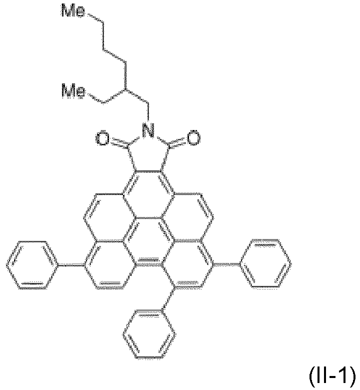 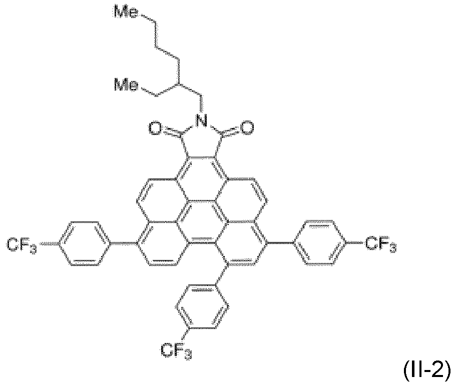 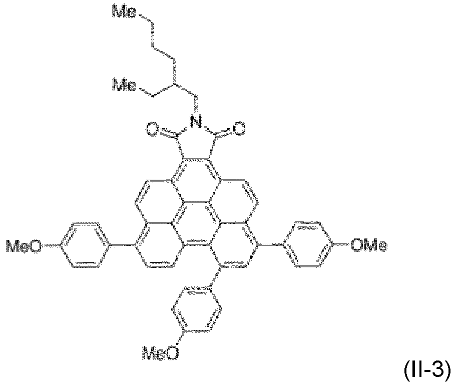
Embodiment {14} relates to the process according to one of the embodiments {01} to {03},
   wherein in Formula (III)
   R¹ is C₁₋₆alkyl;
   R¹⁰ is phenyl which is optionally substituted with one or more substituents being selected independently from each other from C₁₋₆alkyl, C₁₋₆haloalkyl (e.g. C₁₋₆fluoroalkyl) and -O-(C₁₋₆alkyl);
   R² to R⁹ are selected independently from each other from hydrogen and C₁₋₆alkyl.
Embodiment {15} relates to the process according to embodiment {14}, wherein the polycyclic compound is a compound of Formula (III-1): 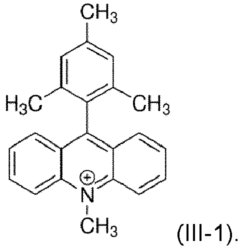
Embodiment {16} relates to the process according to one of the embodiments {01} to {15}, wherein the palladium salt is a palladium acetate, a palladium acetylacetonate, or a palladium halide (such as palladium chloride, palladium bromide or palladium iodide), or any mixture thereof.
Embodiment {17} relates to the process according to one of the embodiments {01} to {15}, wherein the palladium complex comprises one or more ligands which are selected from a phosphine, a 1,4-dien-3-one (e.g. dibenzylideneacetone), an N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene or cyclooctatetraene), an amine, or any combination thereof.
Embodiment {18} relates to the process according to embodiment {17}, wherein the palladium complex additionally comprises one or more ligands which are selected from a halide (e.g. Cl⁻, Br⁻), a pseudohalide (e.g. CN⁻ or OCN⁻), an acetylacetonate and a carboxylate (e.g. acetate). Embodiment {19} relates to the process according to one of the embodiments {01} to {18}, wherein the catalyst composition comprises a component (iii) which is selected from a phosphine, a bipyridine or a cyclic diamine or a mixture of at least two of these compounds.
Embodiment {20} relates to the process according to embodiment {19}, wherein the palladium compound of component (i) is a palladium salt or a phosphine-free palladium complex (such as a phosphine-free palladium complex comprising one or more ligands selected from a 1,4-dien-3-one (e.g. dibenzylideneacetone), a N-heterocyclic carbene, an aryl, a nitrile (e.g. acetonitrile, propionitrile or benzonitrile), a polyene (e.g. a polyene having 2 to 4 carbon-carbon double bonds, in particular a cyclic polyene such as cyclooctadiene (COD) or cyclooctatetraene) and an amine, and optionally comprising one or more additional ligands selected from a halide, a pseudohalide (e.g. CN⁻ or OCN⁻), an acetylacetonate and a carboxylate (e.g. acetate)).
Embodiment {21} relates to the process according to embodiment {19} or {20}, wherein the phosphine of component (iii) is of Formula (IV):

   P(R¹)(R²)(R³) (IV)

   wherein
   R¹ and R² are selected independently from each other from C₁₋₆ alkyl and C₅₋₇-cycloalkyl (e.g. cyclohexyl);
   R³ is biphenyl which is optionally substituted with one or more R⁴;
   R⁴, if present, is C₁₋₆ alkyl, C₁₋₆ alkoxy, phenyl, or pyridyl (more preferably C₁₋₆ alkyl or C₁₋₆ alkoxy).
Embodiment {22} relates to the process according to embodiment {21} wherein the phosphine of Formula (IV) has one of the following Formulas (IV-1) to (IV-6): 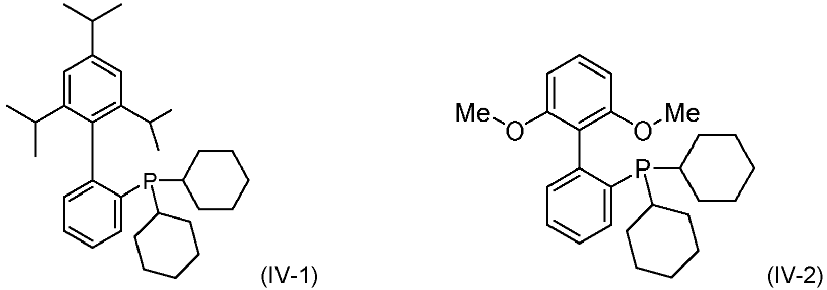 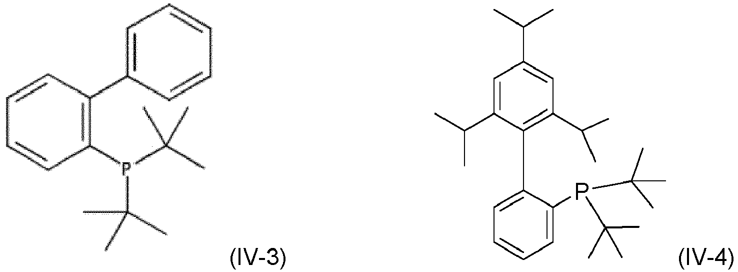 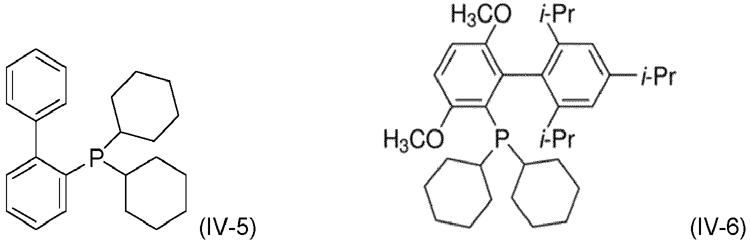
Embodiment {23} relates to the process according to embodiment {19} or {20}, wherein the phosphine of component (iii) is of Formula (V):

   P(R¹)(R²)(R³) (V)

   wherein
   each of R¹ to R³ is C₁₋₆alkyl; or
   each of R¹ to R³ is C₅₋₇cycloalkyl (e.g. cyclohexyl); or
   R¹ to R³ are selected independently from each other from aryl (e.g. phenyl) and heteroaryl (e.g. pyridyl), wherein each aryl or heteroaryl is optionally substituted with one or more C₁₋₄ haloalkyl (e.g. -CF₃), C₁₋₄ alkyl (e.g. methyl) or C₁₋₄ alkoxy (e.g. methoxy).
Embodiment {24} relates to the process according to embodiment {23}, wherein the phosphine of component (iii) has one of the following Formulas (V-1) to (V-6): 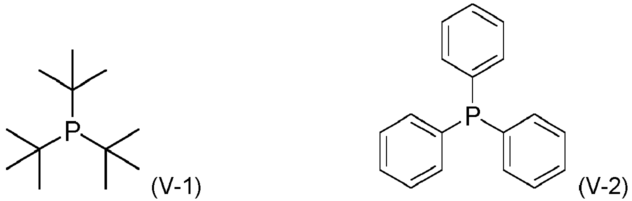 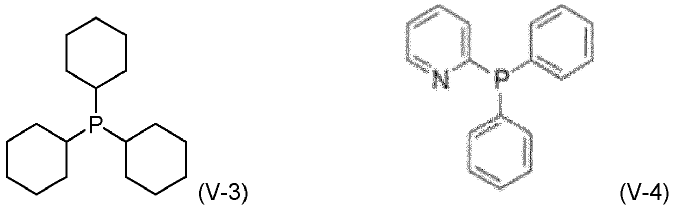 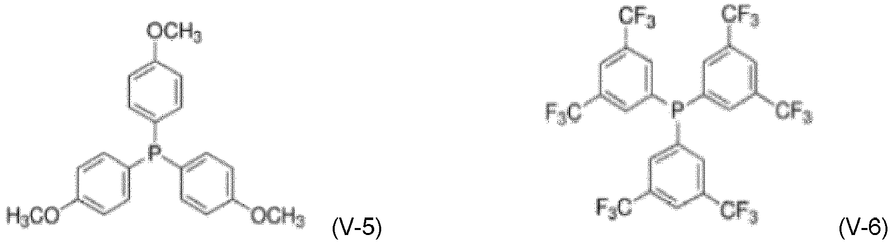
Embodiment {25} relates to the process according to embodiment {19} or {20}, wherein the phosphine of component (iii) is a ferrocenyl diphosphine or a xanthenyl diphosphine.
Embodiment {26} relates to the process according to embodiment {25}, wherein the ferrocenyl diphosphine has one of the following Formulas (VI-1) to (VI-2): 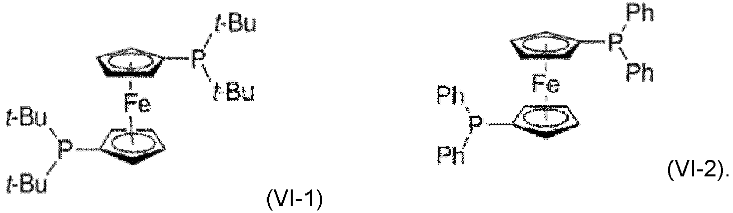
Embodiment {27} relates to the process according to embodiment {25}, wherein the xanthenyl diphosphine has the following Formula (VII-1): 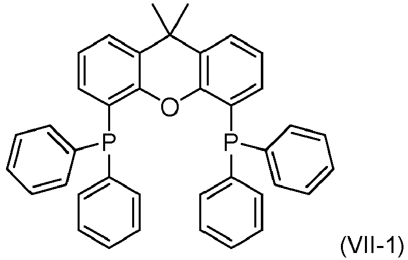
Embodiment {28} relates to the process according to embodiment {19} or {20}, wherein the phosphine of component (iii) is a diphosphine of Formula (VIII):

   (R¹)(R²)P-(CH₂)ₙ-P(R³)(R⁴) (VIII)

   wherein
   n is 1 to 6, more preferably 2 to 4;
   R¹, R², R³ and R⁴ are selected independently from each other from phenyl and cyclohexyl.
Embodiment {29} relates to the process according to embodiment {28}, wherein the phosphine of Formula (VIII) has one of the following Formulas (VIII-1) to (VIII-3): 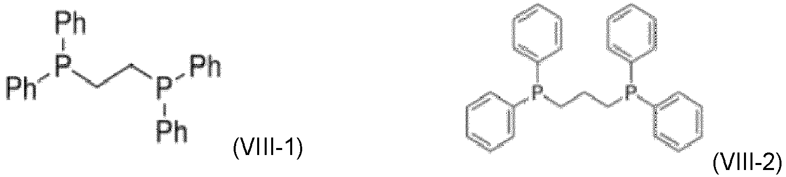 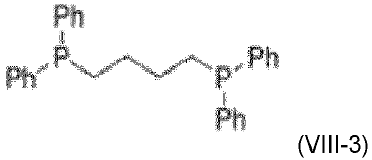
Embodiment {30} relates to the process according to embodiment {19} or {20}, wherein the phosphine of component (iii) is a 2,3-dihydrobenzo[d][1,3]oxaphosphole, which preferably has the following Formula (IX): 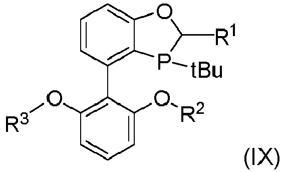 wherein
   R¹ to R³ are selected independently from each other from hydrogen and C₁₋₄alkyl.
Embodiment {31} relates to the process according to embodiment {19} or {20}, wherein the phosphine of component (iii) is a diadamantyl-C₁₋₆alkyl-phosphine, which preferably has the following Formula (X-1): 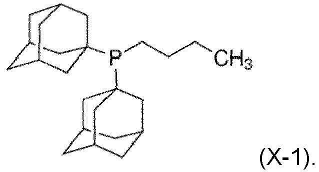
Embodiment {32} relates to the process according to embodiment {19} or {20}, wherein the cyclic diamine of component (iii) is a bicyclic diamine (such as 1,4-diazabicyclo[2.2.2]octane (DABCO)).
Embodiment {33} relates to the process according to one of the embodiments {01} to {32}, wherein the palladium complex is of Formula (XI) 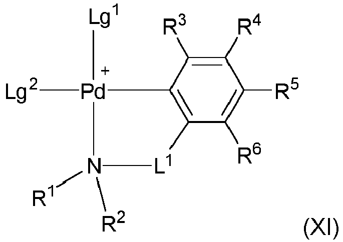 wherein
   L¹ is a bivalent linker;
   R¹ and R² are selected independently from each other from hydrogen and C₁₋₄alkyl; or R¹ and R² together with the nitrogen atom to which they are attached form a five- or six-membered ring (which might be an aromatic or non-aromatic ring);
   R³, R⁴, R⁵, and R⁶ are selected independently from each other from hydrogen and C₁₋₄alkyl; or at least two of the residues R³ to R⁶ which are adjacent to each other form together with the carbon atoms to which they are attached a five- or six-membered aromatic ring and, if present, the remaining residues (i.e. those of the residues R³ to R⁶ which do not form a ring) are selected independently from each other from hydrogen and C₁₋₄alkyl;
   Lg¹ and Lg² are, independently from each other, a non-ionic ligand.
Embodiment {34} relates to the process according to embodiment {33}, wherein
   L¹ is C₁₋₃ alkylene (e.g. -CH₂-; -CH₂-CH₂-; -CH₂-CH₂-CH₂-);
   Lg¹ is a pyridine, a pyrrole, an imidazole, a pyrazole or a pyrimidine, each of which is optionally substituted with one or more halogens;
   Lg² is a N-heterocyclic carbene.
Embodiment {35} relates to the process according to embodiment {34}, wherein the N-heterocyclic carbene is of Formula (XII) or Formula (XIII): 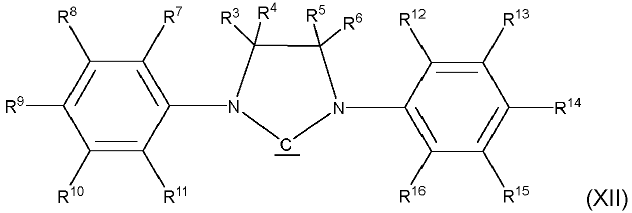
   wherein in Formula (XII)
      R³ to R¹⁶ are selected independently from each other from hydrogen and C₁₋₄alkyl; or at least two of the residues R³ to R¹⁶ which are adjacent to each other form together with the carbon atoms to which they are attached a 5- or 6-membered ring and, if present, the remaining residues (i.e. those of the residues R³ to R¹⁶ not forming a ring) are selected independently from each other from hydrogen and C₁₋₄alkyl; 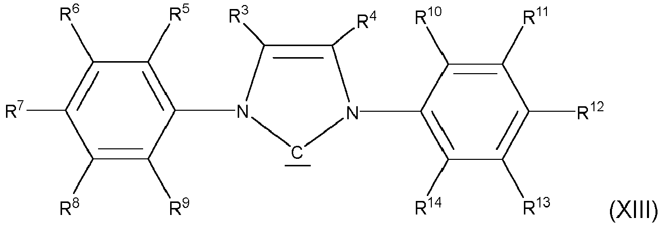
   wherein in Formula (XIII)
      R³ to R¹⁴ are selected independently from each other from hydrogen and C₁₋₄ alkyl; or at least two of the residues R³ to R¹⁴ which are adjacent to each other form together with the carbon atoms to which they are attached a 5- or 6-membered ring and, if present, the remaining residues (i.e. those of the residues R³ to R¹⁴ not forming a ring) are selected independently from each other from hydrogen and C₁₋₄alkyl.
Embodiment {36} relates to the process according to one of the embodiments {33} to {35}, wherein the palladium complex is of Formula (XI-1): 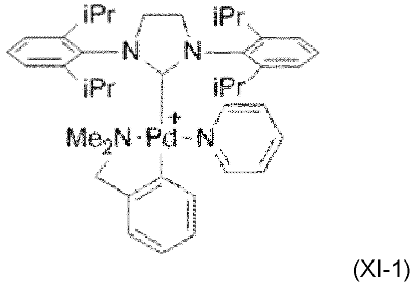
Embodiment {37} relates to the process according to one of the embodiments {01} to {32}, wherein the palladium complex is of Formula (XIV) 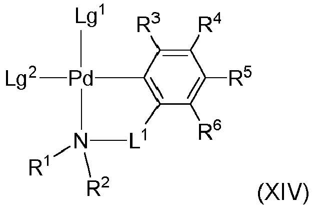 wherein
   L¹ is a bivalent linker;
   R¹ and R² are selected independently from each other from hydrogen and C₁₋₄ alkyl; or R¹ and R² together with the nitrogen atom to which they are attached form a five- or six-membered ring,
   R³, R⁴, R⁵, and R⁶ are selected independently from each other from hydrogen and C₁₋₄ alkyl; or at least two of the residues R³ to R⁶ which are adjacent to each other form together with the carbon atoms to which they are attached a five- or six-membered aromatic ring and, if present, the remaining residues (i.e. those of the residues R³ to R⁶ which do not form a ring) are selected independently from each other from hydrogen and C₁₋₄alkyl,
   Lg¹ is a non-ionic ligand,
   Lg² is an anionic ligand.
Embodiment {38} relates to the process according to embodiment {37}, wherein
   L¹ is phenylene (e.g. 1 ,2-phenylene) or C₁₋₃ alkylene (e.g. -CH₂-; -CH₂-CH₂-; -CH₂-CH₂-CH₂-);
   the non-ionic ligand Lg¹ is a phosphine;
   the anionic ligand Lg² is an alkyl sulfonate (e.g. mesylate) or an aryl sulfonate (e.g. tosylate).
Embodiment {39} relates to the process according to embodiment {38}, wherein the phosphine ligand Lg1 is of Formula (XV):

   P(R¹)(R²)(R³) (XV)

   wherein
   R¹ and R² are selected independently from each other from C₁₋₆ alkyl, cycloalkyl and phenyl, wherein phenyl is optionally substituted with one or more C₁₋₄ alkyl;
   R³ is biphenyl or binaphthyl, each of which is optionally substituted with one or more R⁴;
   R⁴, if present, is C₁₋₆ alkyl, -NH₂, -N(H)(C₁₋₆ alkyl), -N(C₁₋₆ alkyl)₂, -P(Phenyl)₂, or -O-C₁₋₆alkyl.
Embodiment {40} relates to the process according to embodiment {39}, wherein the palladium complex is selected from Formulas (XIV-1) to (XIV-7): 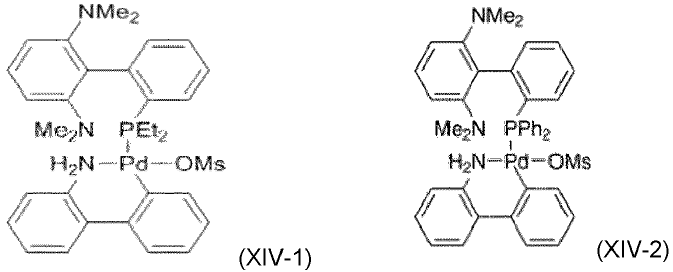 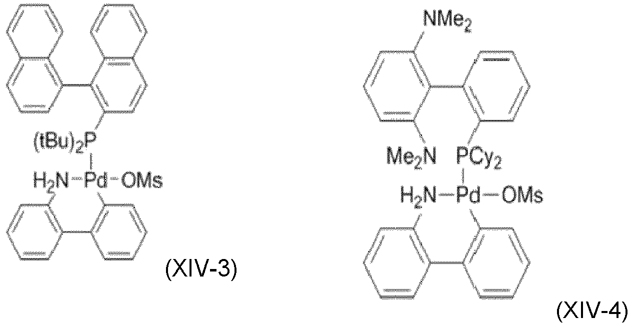 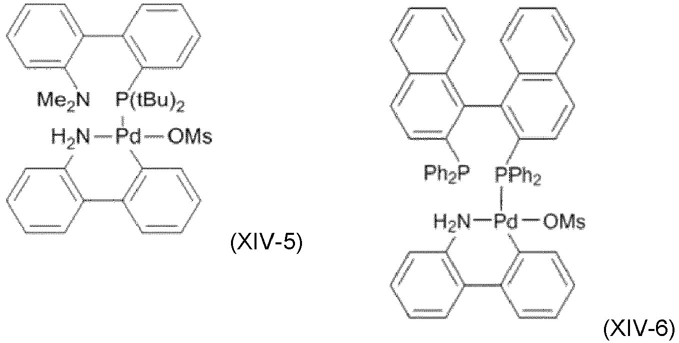 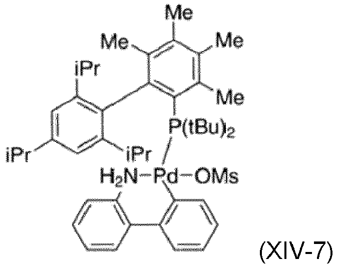
Embodiment {41} relates to the process according to embodiment {38}, wherein the phosphine ligand Lg1 is a ferrocenyl diphosphine.
Embodiment {42} relates to the process according to embodiment {41}, wherein the palladium complex is of Formula (XIV-8) or Formula (XIV-9): 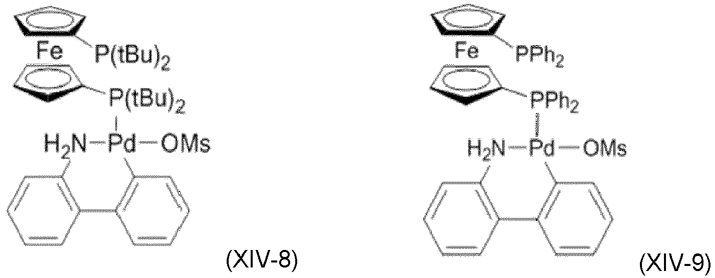
Embodiment {43} relates to the process according to embodiment {38}, wherein the phosphine ligand Lg¹ is a trialkyl phosphine (e.g. P(C₁₋₆ alkyl)₃).
Embodiment {44} relates to the process according to embodiment {43}, wherein the palladium complex is of Formula (XIV-10): 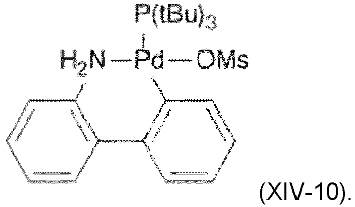
Embodiment {45} relates to the process according to embodiment {38}, wherein the palladium complex is of Formula (XIV-11), (XIV-12), or (XIV-13): 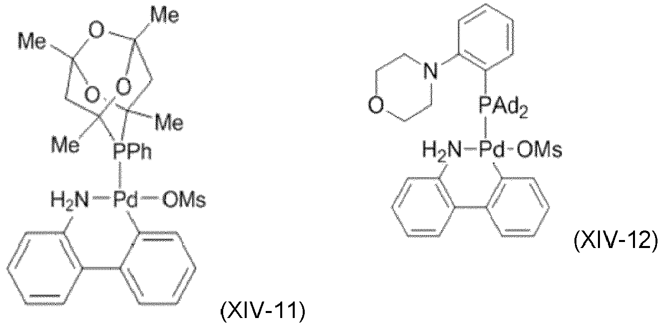 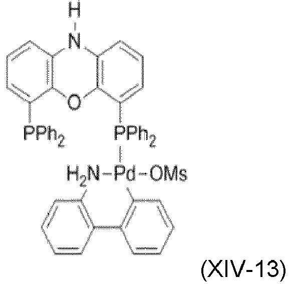
Embodiment {46} relates to the process according to one of the embodiments {01} to {32}, wherein the palladium complex is of Formula (XVI):

   Pd(L)₃₋₄ (XVI)

   wherein
   each of the ligands L is a triaryl phosphine (e.g. a triphenyl phosphine), wherein each aryl group is optionally substituted with one or more R¹;
   R¹, if present, is C₁₋₄ haloalkyl (e.g. -CF₃), C₁₋₄ alkyl (e.g. methyl), C₁₋₄ alkoxy (e.g. methoxy).
Embodiment {47} relates to the process according to embodiment {46}, wherein the palladium complex is of Formula (XVI-1) or (XVI-2): 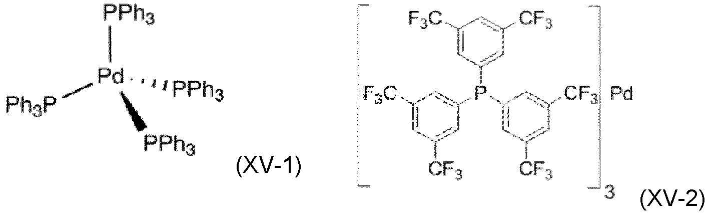
Embodiment {48} relates to the process according to one of the embodiments {01} to {32}, wherein the palladium complex is of Formula (XVII): 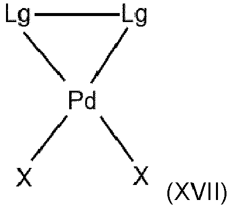 wherein
   Lg-Lg is a chelating ferrocenyl diphosphine ligand,
   X is a halide (e.g. CI).
Embodiment {49} relates to the process according to embodiment {48}, wherein the palladium complex is of Formula (XVII-1) or (XVII-2): 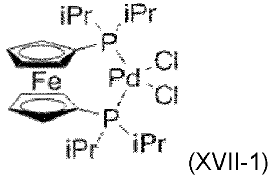 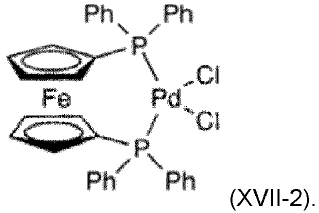
Embodiment {50} relates to the process according to one of the embodiments {01} to {49}, wherein the molar ratio between the palladium compound and the one or more compounds of component (ii) is in the range of from 100/1 to 1/100, more preferably 10/1 to 1/10.
Embodiment {51} relates to the process according to one of the embodiments {01} to {50}, wherein the heterocyclic aromatic ring AR1 is a 5- or 6-membered aromatic ring containing one or more heteroatoms (e.g. up to three heteroatoms) which are selected from nitrogen, oxygen and sulfur, and the carboxylic acid group, carboxylate group or carboxylic acid ester group is attached to a carbon ring atom of the heterocyclic aromatic ring AR1.
Embodiment {52} relates to the process according to one of the embodiments {01} to {51}, wherein the carboxylic acid ester group attached to the heterocyclic aromatic ring AR1 has the following formula (XVIII):

   -C(O)O-A (XVIII)

   wherein the residue A is derived from a N-hydroxy compound, which is preferably selected from an oxime (a ketoximes or an aldoxime), a N-hydroxy phthalimide, a N-hydroxypyridine-2-thione, and a N-hydroxy benzotriazole.
Embodiment {53} relates to the process according to embodiment {52}, wherein the residue A in Formula (XVIII) has one of the following Formulas (XIX-1) to (XIX-6): 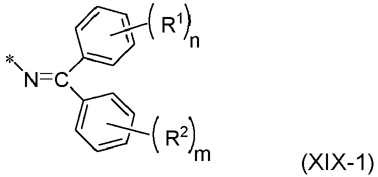 wherein in Formula (XIX-1)
   n is 0 or 1;
   m is 0 or 1;
   R¹ and R² are selected independently from each other from halogen (e.g. Cl or F) and halo-C₁₋₄alkyl (e.g. -CF₃); 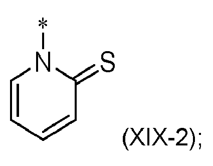 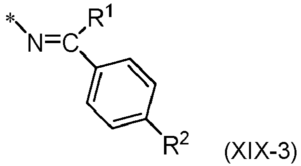
   wherein in Formula (XIX-3)
   R¹ is hydrogen or CF3,
   R² is hydrogen or fluoro; 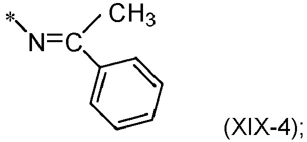 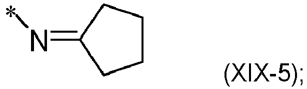 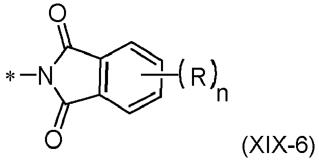
   wherein in Formula (XIX-6)
   n is an integer from 0 to 4,
   R is halogen (e.g. CI).
Embodiment {54} relates to the process according to one of the embodiments {01} to {53}, wherein the reactants and the catalyst composition are mixed with each other in a solvent which comprises a polar aprotic solvent (such as dimethylsulfoxide (DMSO), dimethylformamide (DMF), dimethylacetamide, sulfolane and ethylacetate).
Embodiment {55} relates to the process according to one of the embodiments {01} to {54}, wherein the external light source is a lamp (such as an UV or UV/VIS lamp), a LED, a laser, or any combination of two or more of these external light sources.
Embodiment {56} relates to the process according to one of the embodiments {01} to {55}, wherein the light reaching the reaction medium has an irradiance of 10 to 2000 milliwatts per square centimeter.
Embodiment {57} relates to the use of the catalyst composition described above (i.e. the composition comprising the palladium compound of component (i), the polycyclic compound of component (ii), and optionally the compound of component (iii)) as a catalyst for coupling a heterocyclic aromatic ring AR1 and a carbocyclic or heterocyclic aromatic ring AR2 to each other by a light-assisted decarboxylative carbon-carbon cross-coupling reaction.

## Claims

1. A process which comprises
- mixing a first reactant, a second reactant and a catalyst composition, thereby providing a reaction medium, wherein
the first reactant is a compound which comprises a heterocyclic aromatic ring AR1 to which a carboxylic acid group, a carboxylate group or a carboxylic acid ester group is attached,
the second reactant is a compound which comprises an aromatic ring AR2 to which a halogen atom, a pseudohalogen group or a sulfonate group is attached,
the catalyst composition comprises
(i) a palladium compound which is selected from a palladium salt or a palladium complex or a mixture thereof,
(ii) a polycyclic compound of Formula (I), (II) or (III): 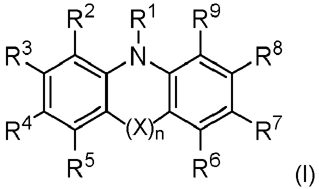 wherein in Formula (I)
n is 0 or 1;
X is O, C(R¹⁰)(R¹¹), or N(R¹²);
R¹ to R¹² are selected independently from each other from hydrogen, aryl and C₁₋₁₀alkyl, wherein aryl and C₁₋₁₀alkyl are optionally substituted with one or more substituents R¹³;
wherein the one or more substituents R¹³, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 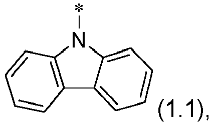 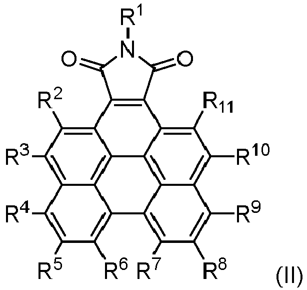
wherein in Formula (II)
R¹ is selected from C₁₋₁₆alkyl and aryl, each of which is optionally substituted with one or more substituents R¹², and/or C₁₋₁₆alkyl is optionally interrupted by one or more oxygen atoms;
R² to R¹¹ are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms; or at least two of the residues R² to R¹¹ which are adjacent to each other form together with the carbon atoms to which they are attached a five- or six-membered aromatic ring and, if present, the remaining residues are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms;
wherein the one or more substituents R¹², if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 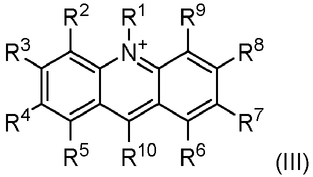
wherein in Formula (III)
R¹ is selected from C₁₋₁₆alkyl and aryl, each of which is optionally substituted with one or more substituents R¹¹, and/or C₁₋₁₆alkyl is optionally interrupted by one or more oxygen atoms;
R² to R⁹ are selected independently from each other from hydrogen, C₁₋₁₆alkyl and aryl, wherein C₁₋₁₆alkyl and aryl are optionally substituted with one or more substituents R¹¹, and/or C₁₋₁₆alkyl is optionally interrupted by one or more oxygen atoms;
R¹⁰ is aryl which is optionally substituted with one or more substituents R¹²; wherein the one or more substituents R¹¹, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl);
wherein the one or more substituents R¹², if present, are selected independently from each other from C₁₋₆alkyl and aryl, wherein aryl is optionally substituted with one or more C₁₋₆alkyl;
- irradiating the reaction medium by an external light source, thereby coupling the heterocyclic aromatic ring AR1 of the first reactant to the aromatic ring AR2 of the second reactant by a decarboxylative carbon-carbon cross-coupling reaction.

2. A process which comprises
- mixing a reactant and a catalyst composition, thereby providing a reaction medium, wherein
the reactant is a compound which comprises a heterocyclic aromatic ring AR1 to which a carboxylic acid group, a carboxylate group or a carboxylic acid ester group is attached and an aromatic ring AR2 to which a halogen atom, a pseudohalogen group or a sulfonate group is attached,
the catalyst composition comprises
(i) a palladium compound which is selected from a palladium salt or a palladium complex or a mixture thereof,
(ii) a polycyclic compound of Formula (I), (II) or (III): 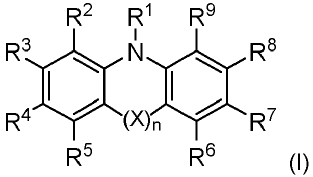 wherein in Formula (I)
n is 0 or 1;
X is O, C(R¹⁰)(R¹¹), or N(R¹²);
R¹ to R¹² are selected independently from each other from hydrogen, aryl and C₁₋₁₀alkyl, wherein aryl and C₁₋₁₀alkyl are optionally substituted with one or more substituents R¹³;
wherein the one or more substituents R¹³, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 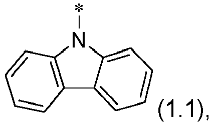 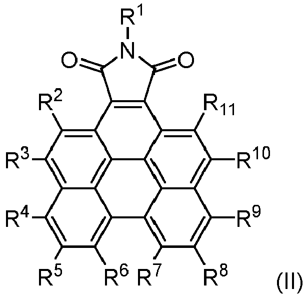
wherein in Formula (II)
R¹ is selected from C₁₋₁₆alkyl and aryl, each of which is optionally substituted with one or more substituents R¹², and/or C₁₋₁₆alkyl is optionally interrupted by one or more oxygen atoms;
R² to R¹¹ are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms; or at least two of the residues R² to R¹¹ which are adjacent to each other form together with the carbon atoms to which they are attached a five- or six-membered aromatic ring and, if present, the remaining residues are selected independently from each other from hydrogen, C₁₋₁₆alkyl, aryl and heteroaryl, wherein C₁₋₁₆alkyl, aryl and heteroaryl are optionally substituted with one or more substituents R¹², and/or C₁₋₁₆alkyl is optionally interrupted by one or more oxygen atoms;
wherein the one or more substituents R¹², if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 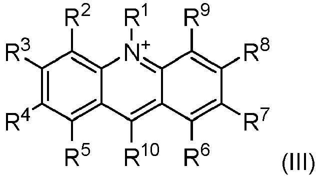
wherein in Formula (III)
R¹ is selected from C₁₋₁₆alkyl and aryl, each of which is optionally substituted with one or more substituents R¹¹, and/or C₁₋₁₆alkyl is optionally interrupted by one or more oxygen atoms;
R² to R⁹ are selected independently from each other from hydrogen, C₁₋₁₆alkyl and aryl, wherein C₁₋₁₆alkyl and aryl are optionally substituted with one or more substituents R¹¹, and/or C₁₋₁₆alkyl is optionally interrupted by one or more oxygen atoms;
R¹⁰ is aryl which is optionally substituted with one or more substituents R¹²;
wherein the one or more substituents R¹¹, if present, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl);
wherein the one or more substituents R¹², if present, are selected independently from each other from C₁₋₆alkyl and aryl (e.g. phenyl), wherein aryl is optionally substituted with one or more C₁₋₆alkyl;
- irradiating the reaction medium by an external light source, thereby coupling the heterocyclic aromatic ring AR1 and the aromatic ring AR2 of the reactant to each other by a decarboxylative carbon-carbon cross-coupling reaction.

3. The process according to claim 1 or 2, wherein R¹ in Formula (I) is selected from phenyl, naphthyl and biphenyl, each of which is optionally substituted with one or more substituents R¹³, wherein R¹³ has the same meaning as in claim 1.

4. The process according to one of the preceding claims, wherein in Formula (I) n is 0;
R¹ is selected from naphthyl, phenyl and biphenyl, each of which is optionally substituted with one or more R¹³;
R² to R⁹ are selected independently from each other from hydrogen, C₁₋₆alkyl, phenyl, biphenyl and naphthyl, wherein phenyl, biphenyl and naphthyl are optionally substituted with one or more substituents R¹³;
wherein, if present, the one or more substituents R¹³, are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1)

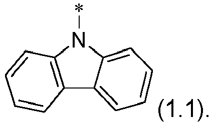

5. The process according to one of the claims 1 to 3, wherein in Formula (I)
n is 1;
X is O;
R¹ is naphthyl which is optionally substituted with one or more substituents R¹³; R², R³, R⁵, R⁶, R⁸ and R⁹ are selected independently from each other from hydrogen and C₁₋₆alkyl;
R⁴ and R⁷ are selected independently from each other from phenyl, biphenyl and naphthyl, each of which is optionally substituted with one or more substituents R¹³; wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl),-C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

6. The process according to one of the claims 1 to 3, wherein in Formula (I)
n is 1;
X is C(R¹⁰)(R¹¹), wherein R¹⁰ and R¹¹ are selected independently from each other from hydrogen and a C₁₋₆alkyl group;
R¹ is selected from naphthyl, phenyl and biphenyl, each of which is optionally substituted with one or more substituents R¹³;
R², R³, R⁵, R⁶, R⁸ and R⁹ are selected independently from each other from hydrogen and C₁₋₆alkyl;
R⁴ and R⁷ are selected independently from each other from phenyl, naphthyl and biphenyl, each of which is optionally substituted with one or more substituents R¹³; wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl) and -S(O)₂(phenyl).

7. The process according to one of the claims 1 to 3, wherein in Formula (I)
n is 1;
Xis N(R¹²);
R¹ and R¹² are selected independently from each other from phenyl, naphthyl and biphenyl, each of which is optionally substituted with one or more substituents R¹³;
R² to R⁹ are selected independently from each other from hydrogen and C₁₋₆alkyl; or at least two of the residues R³, R⁴, R⁷, and R⁸ are phenyl which is optionally substituted with one or more substituents R¹³ and the residues R², R⁵, R⁶ and R⁹ are selected independently from each other from hydrogen and C₁₋₆alkyl;
wherein, if present, the one or more substituents R¹³ are selected independently from each other from C₁₋₆alkyl, C₁₋₆haloalkyl, -OH, -O-(C₁₋₆alkyl), -NO₂, -CN, halogen, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

8. The process according to one of the claims 1 to 3, wherein in Formula (II)
R¹ is C₁₋₁₂alkyl which is optionally substituted with one or more substituents being selected from -OH, halogen, -NH₂, -NH(C₁₋₆alkyl) and -N(C₁₋₆alkyl)₂;
R⁴, R⁷, and R⁹ are selected independently from each other from hydrogen, aryl, heteroaryl, C₁₋₆alkyl and -O-(C₁₋₆alkyl), wherein aryl and heteroaryl are optionally substituted with one or more substituents which are selected independently from each other from C₁₋₆alkyl, C₁₋₆haloalkyl (e.g. C₁₋₆fluoroalkyl) and -O-(C₁₋₆alkyl);
R², R³, R⁵, R⁶, R⁸, R¹⁰ and R¹¹ are selected independently from each other from hydrogen and C₁₋₆alkyl.

9. The process according to one of the claims 1 to 3, wherein in Formula (III)
R¹ is C₁₋₆alkyl;
R¹⁰ is phenyl which is optionally substituted with one or more substituents being selected independently from each other from C₁₋₆alkyl, C₁₋₆haloalkyl and -O-(C₁₋₆alkyl);
R² to R⁹ are selected independently from each other from hydrogen and C₁₋₆alkyl.

10. The process according to one of the preceding claims, wherein the palladium salt is a palladium acetate, a palladium acetylacetonate, a palladium halide, or any mixture thereof.

11. The process according to one of the claims 1 to 9, wherein the palladium complex comprises one or more ligands which are selected from a phosphine, a 1,4-dien-3-one, a N-heterocyclic carbene, an aryl, a nitrile, a polyene, an amine, or any combination thereof.

12. The process according to one of the preceding claims, the catalyst composition further comprising a component (iii) which is selected from a phosphine, a bipyridine or a cyclic diamine or a mixture of at least two of these compounds.

13. The process according to claim 12, wherein the phosphine of component (iii) is selected from one or more of the following compounds:
- a phosphine of Formula (IV):
P(R¹)(R²)(R³) (IV)
wherein
R¹ and R² are selected independently from each other from C₁₋₆ alkyl and C₅₋₇-cycloalkyl;
R³ is biphenyl which is optionally substituted with one or more R⁴;
R⁴, if present, is C₁₋₆ alkyl, C₁₋₆ alkoxy, phenyl or pyridyl;
- a phosphine of Formula (V):
P(R¹)(R²)(R³) (V)
wherein
each of R¹ to R³ is C₁₋₆alkyl; or
each of R¹ to R³ is C₅₋₇cycloalkyl; or
R¹ to R³ are selected independently from each other from aryl and heteroaryl, each of which is optionally substituted with one or more C₁₋₄ haloalkyl, C₁₋₄ alkyl or C₁₋₄ alkoxy;
- a ferrocenyl diphosphine;
- a xanthenyl diphosphine;
- a diphosphine of Formula (VIII):
(R¹)(R²)P-(CH₂)ₙ-P(R³)(R⁴) (VIII)
wherein
n is 1 to 6, more preferably 2 to 4;
R¹, R², R³ and R⁴ are selected independently from each other from phenyl and cyclohexyl;
- a 2,3-dihydrobenzo*[d]*[1,3]oxaphosphole;
- a diadamantyl-C₁₋₆alkyl-phosphine.

14. The process according to one of the preceding claims, wherein the palladium complex of component (i) has one of the following Formulas (XI), (XIV), (XVI) or (XVII): 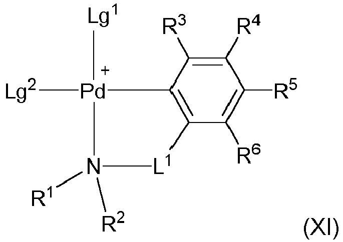 wherein in Formula (XI)
L¹ is a bivalent linker,
R¹ and R² are selected independently from each other from hydrogen and C₁₋₄alkyl; or R¹ and R² together with the nitrogen atom to which they are attached form a five- or six-membered ring;
R³, R⁴, R⁵, and R⁶ are selected independently from each other from hydrogen and C₁₋₄alkyl; or at least two of the residues R³ to R⁶ which are adjacent to each other form together with the carbon atoms to which they are attached a five- or six-membered aromatic ring and, if present, the remaining residues are selected independently from each other from hydrogen and C₁₋₄alkyl,
Lg¹ and Lg² are, independently from each other, a non-ionic ligand; 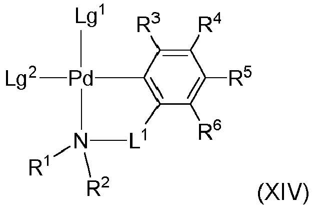
wherein in Formula (XIV)
L¹ is a bivalent linker;
R¹ and R² are selected independently from each other from hydrogen and C₁₋₄ alkyl; or R¹ and R² together with the nitrogen atom to which they are attached form a five- or six-membered ring;
R³, R⁴, R⁵, and R⁶ are selected independently from each other from hydrogen and C₁₋₄ alkyl; or at least two of the residues R³ to R⁶ which are adjacent to each other form together with the carbon atoms to which they are attached a five- or six-membered aromatic ring and, if present, the remaining residues are selected independently from each other from hydrogen and C₁₋₄alkyl;
Lg¹ is a non-ionic ligand;
Lg² is an anionic ligand;
Pd(L)₃₋₄ (XVI)
wherein in Formula (XVI)
the ligands L are independently from each other a triaryl phosphine, wherein each aryl group is optionally substituted with one or more R¹;
R¹, if present, is C₁₋₄haloalkyl, C₁₋₄alkyl or C₁₋₄alkoxy; 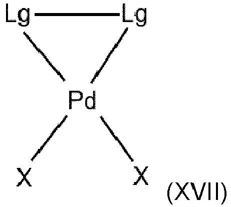
wherein in Formula (XVII)
Lg-Lg is a chelating ferrocenyl diphosphine ligand;
X is a halide.

15. The process according to one of the preceding claims, wherein the carboxylic acid ester group attached to the heterocyclic aromatic ring AR1 has the following formula (XVIII):
-C(O)O-A (XVIII)
wherein the residue A is derived from a N-hydroxy compound.

16. The process according to one of the preceding claims, wherein the reactants and the catalyst composition are mixed with each other in a solvent which comprises a polar aprotic solvent.

17. The process according to one of the preceding claims, wherein the external light source is a lamp (such as a UV or UV/VIS lamp), a LED, a laser, or any combination of two or more of these external light sources.

18. Use of the catalyst composition of one of the claims 1 to 14 as a catalyst for coupling a heterocyclic aromatic ring AR1 and a carbocyclic or heterocyclic aromatic ring AR2 to each other by a light-assisted decarboxylative carbon-carbon cross-coupling reaction.
